# EUROPEAN PATENT SPECIFICATION

(11) **EP 4 038 048 B1**
(45) Date of publication and mention of the grant of the patent: **05.11.2025**
(21) Application number: 20790163.8
(22) Date of filing: 29.09.2020
(51) Int. Cl.: C07C 227/18, C07C 229/08, C07C 229/12, C07C 229/46, C07D 211/00, A61K 8/41, A61Q 11/00

(54) **DERIVATIVES OF MENTHOL AND USES THEREOF**
MENTHOLDERIVATE UND DEREN VERWENDUNGEN
DÉRIVÉS DE MENTHOL ET LEURS UTILISATIONS

(30) Priority: 30.09.2019 US 201962908344 P
(43) Date of publication of application: 10.08.2022
(73) Proprietor: FONA Technologies, LLC, Geneva, IL 60134 (US)
(72) Inventor: KLUMPP, Douglas, A., DeKalb, IL 60115 (US); KLUMPP, Rachel, A., DeKalb, IL 60115 (US); LIVERIS, Zachary, DeKalb, IL 60115 (US); STENTZEL, Michael, DeKalb, IL 60115 (US); SOBEL, Robert, M., Geneva, IL 60134 (US); KOKKINIDOU, Smaro, G., Geneva, IL 60134 (US)
(74) Representative: Müller, Christian Stefan Gerd
(86) International application number: PCT/US2020/053323
(87) International publication number: WO 2021/067301

(56) References cited:
- RIGHETTI ET AL: "Sur les dialcoylbétaïno-acétates de menthyle-1 [l-Menthyl dialkylbetaine acetates]", BULLETIN DE LA SOCIETE CHIMIQUE DE FRANCE. MEMOIRES, MASSON, PARIS, FR, no. 5, 1 January 1938 (1938-01-01), pages 1463 - 1472, XP009524650, ISSN: 0366-3132
- T.S. RAIKOVA ET AL: "Synthesis and structure of amino esters of menthol", CHEMISTRY OF NATURAL COMPOUNDS, vol. 17, 1 January 1981 (1981-01-01), pages 526 - 530, XP055760550
- MARIIA NESTERKINA ET AL: "Repellent Activity of monoterpenoid esters with neurotrnasmitter amino acids against yellow fever mosquito, Aedes aegypti", OPEN CHEMISTRY, vol. 16, no. 1, 9 March 2018 (2018-03-09), pages 95 - 98, XP055760710, DOI: 10.1515/chem-2018-0015
- HIDEKI ISHII ET AL: "Chiral recognition of cyclic [alpha]-hydroxyketones by CD-sensitive zinc tetraphenylporphyrin tweezer", CHIRALITY., vol. 17, no. 6, 1 January 2005 (2005-01-01), US, pages 305 - 315, XP055761462, ISSN: 0899-0042, DOI: 10.1002/chir.20166
- JEAN-CLAUD FIAUD ET AL: "Synthese asymetrique d'acides amines. Etude de l'addition d'organometalliques sur les imines glyoxyliques.", TETRAHEDRON LETTERS, vol. 12, no. 15, 1 January 1971 (1971-01-01), Amsterdam , NL, pages 1019 - 1022, XP055761473, ISSN: 0040-4039, DOI: 10.1016/S0040-4039(01)96614-6
- ECCLES R: "REVIW: MENTHOL AND RELATED COOLING COMPOUNDS", PHARMACEUTICAL AND CLINICAL RESEARCH, JOHN WILEY & SONS LTD, LONDON, GB, vol. 46, 1 January 1994 (1994-01-01), pages 618 - 630, XP009076858, ISSN: 0022-3573

## Description

### BACKGROUND

Natural menthol is known as an agent that provides a cooling sensation ("cooling agent") and it has been used for many years as an ingredient in various consumer products, such as candies and gum, toothpaste, mouthwash, shaving cream, and skin ointments. Menthol's ability to chemically trigger the cold-sensitive TRPM8 receptors is responsible for the well-known cooling sensation it provokes when inhaled, eaten, or applied to the skin. TRPM8 is an ion channel, which, upon activation allows the entry of Na⁺ and Ca²⁺ ions to the cell that leads to depolarization and the generation of an action potential. Application of menthol to skin or mucus membranes results directly in membrane depolarization, followed by calcium influx via voltage-dependent calcium channels, providing evidence for the role of TRPM8 and other TRP receptors to mediate our sensory interaction with the environment in response to cold in the same way as in response to menthol. In taste dilution tests, (-)-menthol exhibits a cooling threshold at 0.8 ppm.

### (-)-menthol chemical structure

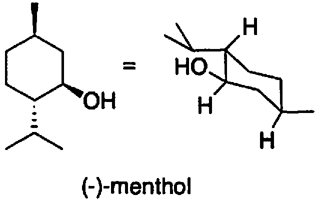

For several decades, there has been an interest in preparing organic compounds which rival or exceed menthol in its perceived cooling sensation. Most notably, a series of Wilkinson Sword compounds (WS) were developed and several have been used in consumer products. Since these developments in the 1970s, a variety of other cooling agents have been proposed and developed - some of which exceed the cooling potential of menthol >200 times. Several cooling agents are known to have carboxyl groups attached to menthol, such as WS-3, WS-5, WS-12, Frescolat ML, and compound **1** (estimated to be ~1000 times cooler than menthol).

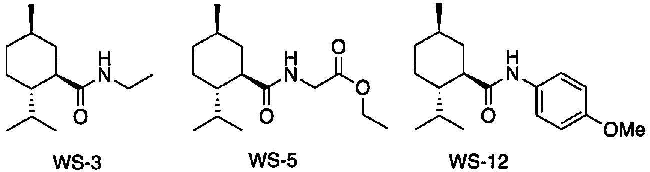

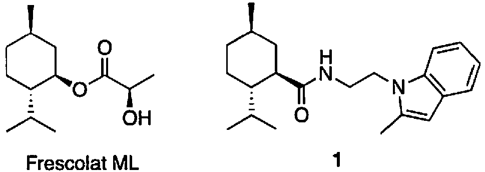

A Chinese patent application (CN 1915966) describes the menthol derivatives N, N-dimethylglycine menthol ester, N, N-diethylglycine menthol ester, and N, N-dihydroxyethylglycine menthol ester, for use as a transdermal agent to promote the subcutaneous absorption of transdermal agents. The Chinese application describes the use of these menthol derivatives in ointments, creams, patches, medicines, and cosmetics.

### SUMMARY

In a first aspect, the application describes a compound of Formula (I): 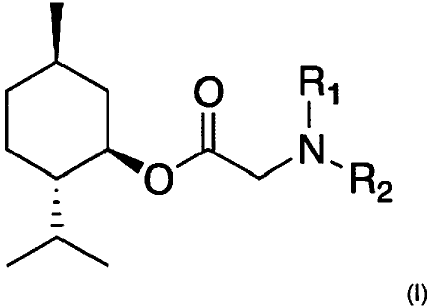 wherein R₁ and R₂ are independently selected from the group consisting of: H, alkyl and R₁ and R₂, together with the N atom to which they are attached, form a 4-8-member ring. R₁ and R₂ each comprise at most 20 carbon atoms and the compound is not N, N-dimethylglycine menthol ester, N, N-diethylglycine menthol ester, or N, N-dihydroxyethylglycine menthol ester.

In a second aspect, the application describes a method for providing a cooling sensation in the mouth, comprising: orally administering a cooling agent, wherein the cooling agent is Formula (I) 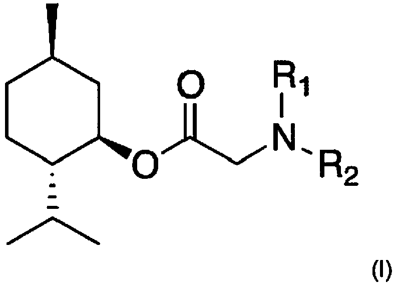 wherein R₁ and R₂ are independently selected from the group consisting of: H, alkyl and R₁ and R₂ together with the N atom to which they are attached form a 4-8-member ring, and R₁ and R₂ each contain at most 20 carbon atoms.

In a third aspect, the application describes a method of making menthol glycinates. The method includes reacting menthol and bromoacetyl bromide or chloroacetyl chloride to form an ester, isolating the ester, and reacting the ester and an amine to form the menthol glycinates.

In a fourth aspect, the application describes an oral hygiene product or an edible product, comprising: a compound of Formula (I) 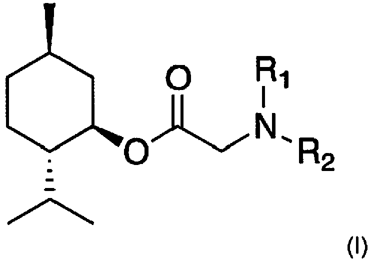 and a solvent or carrier, wherein R₁ and R₂ are independently selected from the group consisting of: H, alkyl and R₁ and R₂ together with the N atom to which they are attached form a 4-8-member ring, and R₁ and R₂ each contain at most 20 carbon atoms.

In a fifth aspect, the application describes an insect repellant, comprising: Formula (I) 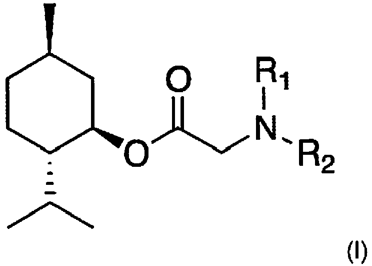 and a carrier. R₁ and R₂ are independently selected from the group consisting of: H, alkyl and R₁ and R₂ together with the N atom to which they are attached form a 4-8-member ring, and R₁ and R₂ each contain at most 20 carbon atoms.

In a sixth aspect, the application includes a compound comprising: Formula (II) 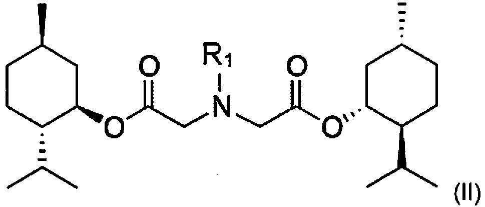 or Formula (III) 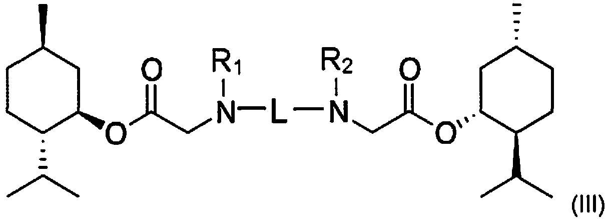 wherein R₁ and R₂ are independently selected from the group consisting of: H, alkyl and R₁ and R₂ together with the N atom to which they are attached form a 4-8-member ring, R₁ and R₂ each contain at most 20 carbon atoms, and L is an alkylene group, arylene group, -(CH₂)_{X}-(OCH₂CH₂)ₙO-(CH₂)_{X}- where x and n are individually 1 to 10 and x + n is at most 10, or 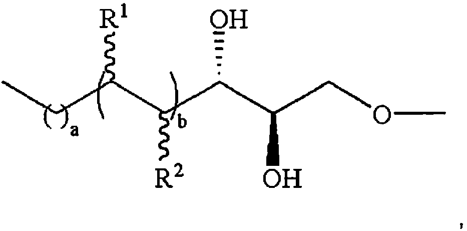 where R¹ and R² are each individually selected from the group consisting of H and OH and a and b are individually 0, 1, 2 or 3, where the L group has at most 20 carbon atoms.

### DEFINITIONS

An aromatic ring or aryl group refers to a monovalent aromatic carbocyclic or heteroaromatic group, preferably of 3 to 10 carbon atoms. The aromatic ring or aryl group can be monocyclic (for example, phenyl (or Ph)) or polycyclic (for example, naphthyl) and can be unsubstituted or substituted. Preferred aryl groups include phenyl, naphthyl, furyl, thienyl, pyridyl, indolyl, quinolinyl or isoquinolinyl.

Alkyl (or alkyl- or alk-) refers to a monovalent, substituted or unsubstituted, saturated or unsaturated, straight, branched or cyclic hydrocarbon chain, preferably containing from 1 to 20 carbon atoms. More preferred alkyl groups are alkyl groups containing from 7 to 16 carbon atoms. Preferred cycloalkyls have from 3 to 10, preferably 3 to 6, carbon atoms in their ring structure. Suitable examples of unsubstituted alkyl groups include methyl, ethyl, propyl, isopropyl, cyclopropyl, butyl, iso-butyl, tert-butyl, sec-butyl, cyclobutyl, pentyl, cyclopentyl, hexyl, and cyclohexyl. Alkylaryl and alkylheterocyclic groups are alkyl groups covalently bonded to an aryl or heterocyclic group, respectively. Unsaturated alkyl refers to alkyl groups containing one or more double and/or triple bonds.

"Substituted" means that the moiety contains at least one, preferably 1-3, substituent(s). Suitable substituents include hydroxyl (-OH), amino (-NH₂), oxy (-O-), carbonyl (-CO-), thiol, alkyl, alkoxy, halo, nitrile, nitro, aryl and heterocyclic groups. These substituents can optionally be further substituted with 1-3 substituents. Examples of substituted substituents include carboxamide, alkylmercapto, alkylsulphonyl, alkylamino, dialkylamino, carboxylate, alkoxycarbonyl, alkylaryl, aralkyl, alkylheterocyclic, and the like.

An arylene group refers to a divalent aromatic carbocyclic or heteroaromatic group, preferably of 3 to 10 carbon atoms. The arylene group can be monocyclic (for example, phenylene (or Ph)) or polycyclic (for example, naphthylene) and can be unsubstituted or substituted. Preferred arylene groups include phenylene, naphthylene, furylene, thienylene, pyridylene, indolylene, quinolinylene or isoquinolinylene.

An alkylene group refers to a divalent, substituted or unsubstituted, saturated or unsaturated, straight, branched or cyclic hydrocarbon chain, preferably containing from 1 to 20 carbon atoms. More preferred alkylene groups are alkylene groups containing from 7 to 16 carbon atoms. Preferred cycloalkylenes have from 3 to 10, preferably 3 to 6, carbon atoms in their ring structure. Suitable examples of unsubstituted alkylene groups include methylene, ethylene, propylene, isopropylene, cyclopropylene, butylene, iso-butylene, tert-butylene, sec-butylene, cyclobutylene, pentylene, cyclopentylene, hexylene, and cyclohexylene.

### BRIEF DESCRIPTION OF THE DRAWINGS

FIG. 1 is a graph showing the average area ratio (Comp./IS) of **XI-1-60** and menthol in ethanol, measured at an initial time, after 24 hours, after 48 hours, after 1 week and after 2 weeks.
FIG. 2 is a graph showing the average area ratio (Comp./IS) of **XI-1-60** and menthol in propylene glycol (PG), measured at an initial time, after 24 hours, after 48 hours, after 1 week and after 2 weeks.
FIG. 3 is a graph showing the average area ratio (Comp./IS) of **XI-1-60** and menthol in water, measured at an initial time, after 24 hours, after 48 hours, after 1 week and after 2 weeks.
FIG. 4 is a graph showing the average area ratio (Comp./IS) of **XI-1-50** and menthol in ethanol, measured at an initial time, after 24 hours, after 48 hours, after 1 week and after 2 weeks.
FIG. 5 is a graph showing the average area ratio (Comp./IS) of **XI-1-50** and menthol in PG, measured at an initial time, after 24 hours, after 48 hours, after 1 week and after 2 weeks.
FIG. 6 is a graph showing the average area ratio (Comp./IS) of **XI-1-50** and menthol in water, measured at an initial time, after 24 hours, after 48 hours, after 1 week and after 2 weeks.
FIG. 7 is a graph showing the average area ratio (Comp./IS) of **RK-2-10** and menthol in ethanol, measured at an initial time, after 24 hours, after 48 hours, after 1 week and after 2 weeks.
FIG. 8 is a graph showing the average area ratio (Comp./IS) of **RK-2-10** and menthol in PG, measured at an initial time, after 24 hours, after 48 hours, after 1 week and after 2 weeks.
FIG. 9 is a graph showing the average area ratio (Comp./IS) of **RK-2-10** and menthol in water, measured at an initial time, after 24 hours, after 48 hours, after 1 week and after 2 weeks.
FIG. 10 is a graph showing the average area ratio (Comp./IS) of menthol in ethanol, measured at an initial time, after 24 hours, after 48 hours, after 1 week and after 2 weeks.
FIG. 11 is a graph showing the time versus intensity for the cooling sensation of menthol and various menthol glycinates in a lozenge.
FIG. 12 is a graph showing the time versus intensity for the cooling sensation of menthol and various menthol glycinates in a beverage model system.
FIG. 13 is a graph showing the time versus intensity for the cooling sensation of menthol and various menthol glycinates in a mouthwash.

### DETAILED DESCRIPTION

The present application describes menthol glycinates, their use as cooling agents, and methods of making the menthol glycinates. Much of the experimental data in this application was published in Klumpp, D. A. et al., "Synthesis of Menthol Glycinates and Their Potential as Cooling Agents.", ACS Omega, vol. 5, no. 8, pp. 4043-4049 (2020). Preferably the menthol glycinate is an ester that includes a menthol connected to a glycine or glycine derivative, via an ester linkage. These menthol glycinates are well suited to provide a prolonged cooling effect, and act as flavoring in food products. Esters hydrolyze at a slow rate in an aqueous environment, providing a slow release of menthol and glycine or a glycine derivative. As the hydrolysis reaction proceeded, more menthol is available to the TRPM8 receptors, providing a cooling effect over a longer period of time. The compounds may also provide a delay in the onset of the cooling. Taste tests have indicated that these menthol glycinates provide a prolonged cooling effect, at a minimum concentration, which far exceeds menthol itself. Additionally, glycine provides a sweet taste and acts as a flavor enhancer, and it is believed that glycine enhances the cooling sensation of the menthol. The amine functional group of the glycine may impart favorable solubility characteristics to the menthol glycinates, making these compounds easier to include in various food products. The menthol glycinates have the following formulas (I), (II) and (III).

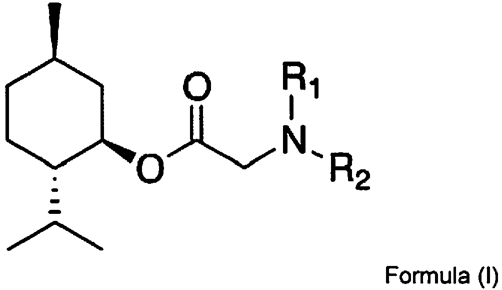

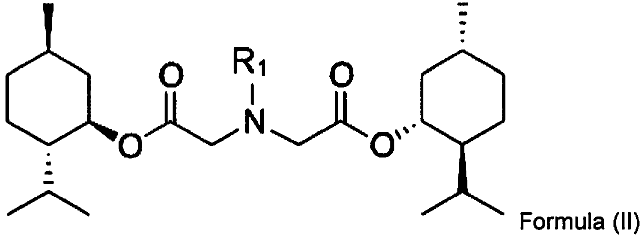

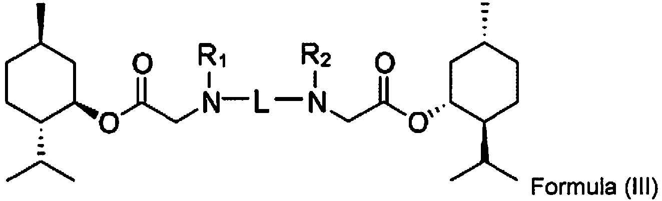

Formula (I) includes a menthol group joined to a glycine derivative, via an ester linkage. The menthol glycinate may also be referred to as a menthol glycinate ester. Formula (II) and Formula (III) illustrate a menthol glycinate, where two menthol groups are included. The R₁ and R₂ may independently be hydrogen or alkyl group. The R₁ and R₂ comprises at most 20 carbon atoms each. R₁ and R₂ may be different or the same. R₁ and R₂ may also form a ring, with the nitrogens as part of the ring. The ring may be, for example, a 4, 5, 6, 7 or 8 membered-ring.

Examples of R₁ and R₂ include methyl, ethyl, propyl, butyl, cyclopropyl, ethyl methyl ether, isopentyl, benzyl, isopropyl, isobutyl, tert-butyl and ethyl pyridine. Examples where R₁ and R₂ form a ring that includes the nitrogen atom include pyrrolidine and piperidine rings.

The L group in Formula (III) is an alkylene group, arylene group, -(CH₂)_{X}-(OCH₂CH₂)ₙO-(CH₂)_{X}- where x and n are individually 1 to 10 and x + n is at most 10, or 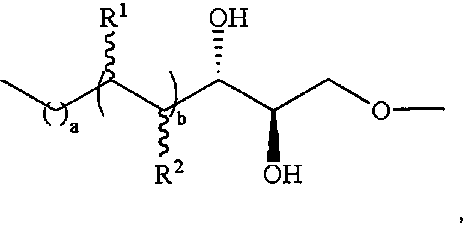 where R¹ and R² are each individually selected from the group consisting of H and OH and a and b are individually 0, 1, 2 or 3, where the L group has at most 20 carbon atoms. Examples of L include (CH₂)_{X} where X=1 to 20, such as methylene, (CH₂)₂, (CH₂)₃, (CH₂)₄, (CH₂)(CH)(CH₃), and (CH₂)(CH₂)(CH)(CH₃).

Methods of producing the desired menthol glycinates include first reacting menthol with bromoacetyl bromide or a similar acid bromide, followed by reacting the product with an amine to form the compounds of interest. The bromide enables easy substitution with a variety of amines, including ammonia. Chloroacetyl chloride may also be used to react with the menthol to produce an ester of chloroacetic acid. The substituents on the amino group may be prepared through simple substitution chemistry. Scheme 1 below illustrates the first step of reacting menthol with bromoacetyl bromide to form product 2. Product 2 may subsequently be reacted with various amines to produce the desired products.

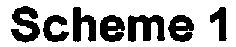

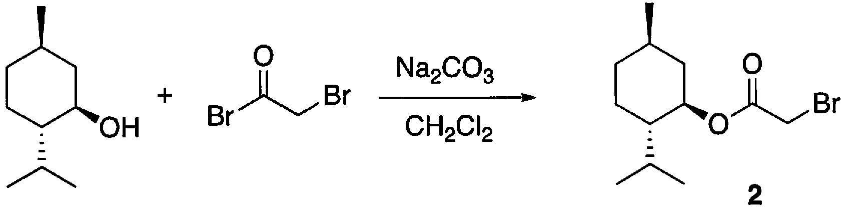

A variety of menthol glycinates may be prepared from the ester of bromoacetic acid and an amine. In general, secondary amines have been found to give the desired substitution products in good yields. Most conversions go to 100% if a slight excess of amine is used.

Menthol glycinates would have the same or similar uses as menthol, and provide a similar cooling sensation. Menthol glycinates may be included in toothpaste, mouthwash, candy, gum, mints, skin creams, aftershave products, smoking products, insect repellants, and as flavoring and perfumes. Menthol glycinates may be used to provide a cooling sensation, to provide a flavoring, to relieve minor sore throats or minor mouth or throat irritation, to reduce itching of skin, to relieve minor aches and pains, to treat sunburns, to remedy bad-breath and as a fragrance to emphasize floral notes.

The menthol glycinates may be included in a formulation including a solvent or carrier, to dissolve, dilute, carry, disperse or deliver the menthol glycinate. Such solvents or carriers include water, castor oil, citric acid esters of mono- and diglycerides, ethyl acetate, glycerol (glycerin), glyceryl diacetate, isopropyl alcohol, monoglycerides and diglycerides, and propylene glycol mono-esters and diesters of fatty acids. For example, gum including the menthol glycinate may also include gum base, sugars, plasticizers, flavors, colors, and/or polyols. Insect repellant may include water, glyceryl stearate, beeswax, vegetable glycerin, xanthan gum, potassium sorbate, and/or citric acid.

The effective concentrations of the menthol glycinates described herein may be determined by routine experimentation. For example, concentration ladders may be used to determine the concentration necessary for the desired result. Different uses may necessitate different concentrations to achieve the desired result. When the menthol glycinates are used as a flavoring the concentration may be, for example, from 1000 ppm to 0.1 ppm. Preferably the concentration is 100 to 0.5 ppm, including 90, 80, 70, 60, 50, 40, 30, 20, 10, 9, 8, 7, 6, 5, 4, 3, 2, 1, 0.9, 0.8, 0.7, and 0.6 ppm.

### EXAMPLES

### Chemicals and materials synthesis

The ester of bromoacetic acid (compound 2 below) is prepared in quantitative yield from menthol and bromoacetyl bromide optionally using sodium carbonate as a base. Dichloromethane may be used as the solvent; however, other solvents may also be used. Isolation of the product involves simple filtration of the solution (to remove the sodium bicarbonate) and evaporation of the solvent under reduced pressure (Scheme 1). Compound 2 is well suited for further synthetic elaboration as the bromomethyl group is reactive towards substitution.

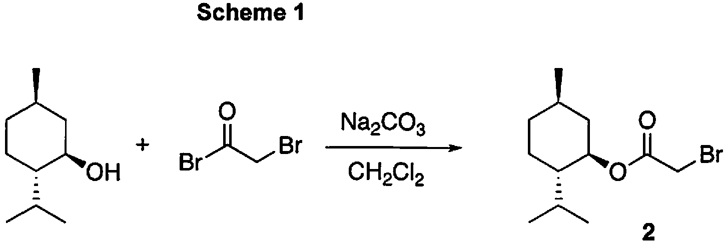

A series of menthol glycinates have been prepared from the ester of bromoacetic acid (see Scheme 2 and Scheme 3). In general, primary and secondary amines have been found to give the desired substitution products in good yields. An optimized procedure with diethylamine provided 95% conversion to 3 with a small amount of unreacted bromide **2.** NMR and GC-FID indicate that compound **3** is minimum 99% pure. Other secondary amines provide the expected substitution products **(4, 5** and **6).** Most conversions go to 100% if a slight excess of amine is used. The primary amine, isopropyl amine, is used to provide compound **7 (DK-2-39** and **DK-1-60** are research notebook references used as alternative compound identifiers for compound 7). The chemistry may also be accomplished using dichloromethane or ether as solvent and sodium carbonate or sodium hydroxide as the base.

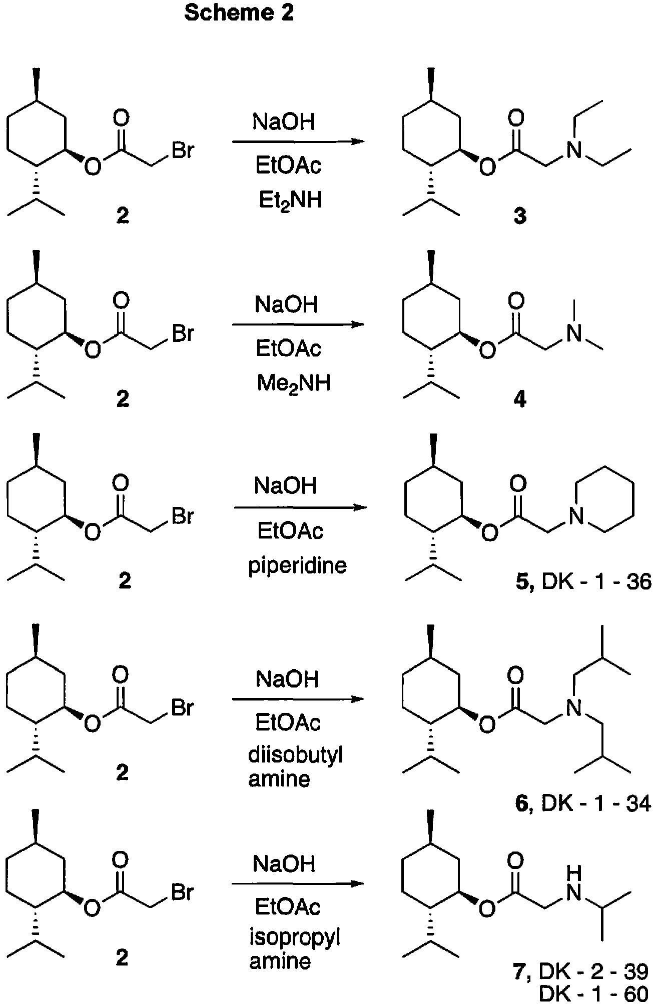

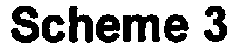

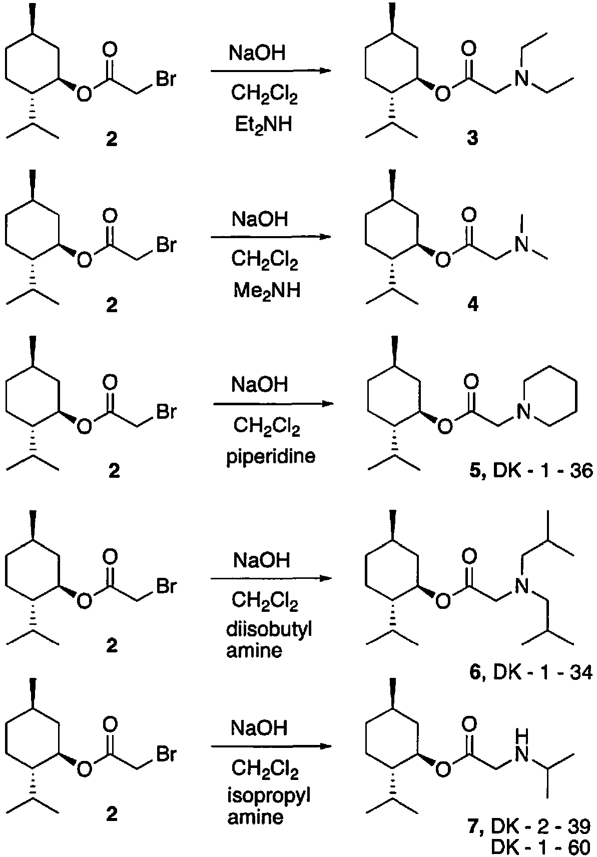

A synthetic procedure has been developed in which compound 4 was prepared in high yield, utilizing only filtrations and solvent removal in reaction workup. Product purification was done using vacuum distillation. Distillation of compound 4 effectively removed any unreacted starting materials of dimethyl amine and the bromo ester (compound 2). The ease of distillation will facilitate the synthesis of large quantities of purified amino ester products.

A variety of secondary amines provided the corresponding menthol glycinates. This includes the dialkylamines to give products **3, 6, 9, 10, 15,** and **16** (see Table 1 below). Heterocyclic systems, such as pyrrolidine and piperidine, were also found to give the substitution products (11 and 5, respectively) in good yields. Primary amines also give the expected substitution products **7, 12, 14,** and **17.** The synthetic method is amenable to incorporating structural components such as cycloalkyl groups, benzyl groups, ethers, and heterocycles. In the case the pyridyl derivative **18,** this compound is modelled after the known cooling agent, amide **19** (FEMA 4549), which is estimated to be about 100 times cooler than menthol.

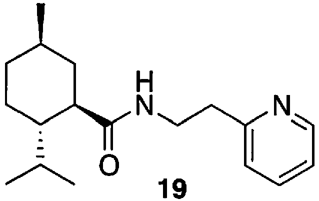

**Table 1: Products and yields from substitution reactions of Compound 1 with amines or ammonia.**

| Product | Yield | Product | **Yield** |
|---|---|---|---|
| 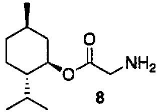 | 80% | 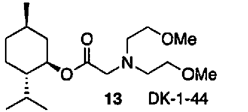 | 68% |
| 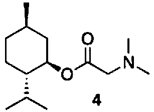 | 86% | 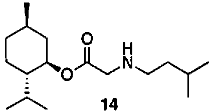 | 83% |
| 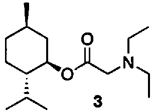 | 92% | 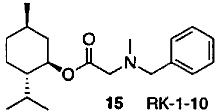 | 90% |
| 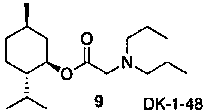 | 81% | 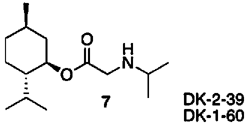 | 88% |
| 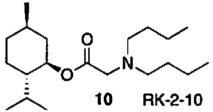 | 79% | 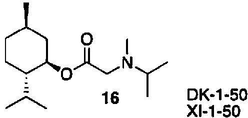 | 81% |
| 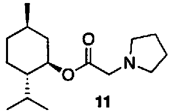 | 81% | 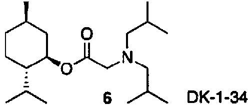 | 86% |
| 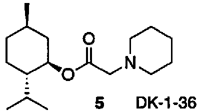 | 61% | 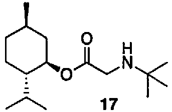 | 88% |
| 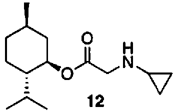 | 74% | 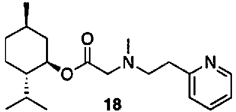 | 75% |

In addition to monosubstitution, products may be prepared from double substitution reactions. Isopropyl amine reacts twice when an excess of compound **2** is used and product **20** is isolated. Similarly, *N,N*'-dimethylethylene diamine reacts twice with compound **2** to provide product **21.** Both **20** and **21** are purified by removal of excess compound **2** via distillation, followed by purification using silica gel chromatography.

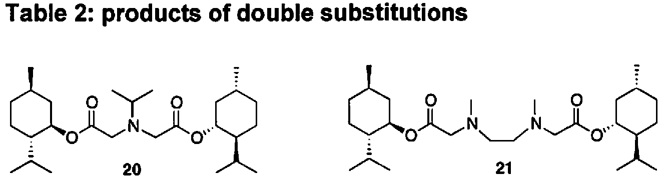

For most of the substitution reactions described above, the optimized procedure involves using an excess of the amine nucleophile. The menthol glycinate products are isolated in pure form by removing the ethyl acetate solvent and excess amine by reduced pressure. Then the menthol glycinate product is typically distilled at 150-220 °C at 1 mm Hg. Optimization studies revealed that excess amine allows the substitution chemistry to go to completion within a relatively short amount of time. For example, dibutylamine was reacted with product 2 of scheme 1, to provide product **10,** and with 1.1 equivalents of the amine, the substitution reaction is only 79% complete after 3 hours. If the amount of dibutylamine is increased to 1.6 equivalents (0.13 M in ethyl acetate), then the substitution reaction is 100% complete in less than 3 hours.

### Preparation of (1R,2S,5R)-2-isopropyl-5-methylcyclohexyl 2-bromoacetate, compound 2.

To 100 mL of CH₂Cl₂, (L)-menthol (7.2 g, 0.046 mmol) is dissolved and anhydrous Na₂CO₃ is added. The resulting solution is cooled to 0 °C and the flask is fitted with a CaCl₂ drying tube. Bromoacetyl bromide (4.0 mL, 0.046 mol) is then added, the cooling bath is removed, and the solution is stirred a minimum of four hours. Following the reaction period, the solution is filtered through glass wool and the solvent removed by vacuum. The product is isolated as a clear colorless oil (12.2 g, 0.044 mol, 96%). Analysis of the crude product by GC-FID and NMR shows an extremely high purity of product, however, the oil may be further refined by distillation (ca. 120 °C, 2 mm). With repeated runs, yields of the crude product varies from 95-100%. ¹H NMR (300 MHz, CDCl₃) δ, 0.78 (d, 3H, J = 6.9 Hz), 0.84-0.96 (m, 7H), 0.96-1.14 (m, 2H), 1.40-1.58 (m, 2H), 1.64-1.74 (m, 2H), 1.88-1.97 (m, 1H), 1.98-2.07 (m, 1H), 3.80-3.82 (m, 2H), 4.70-4.79 (m, 1H). ¹³C NMR (75 MHz, CDCl₃) δ, 16.2, 20.7, 22.0, 23.3, 26.1, 26.3, 31.4, 34.1, 40.5, 46.9, 76.5, 166.9. Low resolution MS (electron impact): 197 (M-80), 141, 139, 138 (100), 123, 109.

### General procedure for the synthesis of menthol glycinates (1° amines).

The amine (0.02 mmol) is dissolved in 25 mL of EtOAc after which is added NaOH (0.6 g, 15 mmol) and anhydrous sodium sulfate (0.5 g, 3.5 mmol). To this solution, the (1R,2S,5R)-2-isopropyl-5-methylcyclohexyl 2-bromoacetate (compound 2, 1.68 g, 6.06 mmol) is slowly added. The mixture is stirred 6 hours or until GC-FID analysis shows no remaining bromoester (2). The solution is then filtered through a plug of glass wool and the solvent removed by reduced pressure. For low boiling amines, the excess amine is removed during this step. For less volatile amines, fractional distillation may be necessary. Final purification of the menthol glycinate is achieve by vacuum distillation.

### General procedure for the synthesis of menthol glycinates (2° amines).

The (1R,2S,5R)-2-isopropyl-5-methylcyclohexyl 2-bromoacetate (compound 2, 1.68 g, 6.06 mmol) is dissolved in 25 mL of EtOAc. To this solution, the amine (9 mmol) is added, followed by NaOH (0.6 g, 15 mmol) and anhydrous sodium sulfate (0.5 g, 3.5 mmol). The mixture is stirred 6 hours or until GC-FID analysis shows no remaining bromoester (2). The solution is then filtered through a plug of glass wool and the solvent removed by reduced pressure. For low boiling amines, the excess amine is removed during this step. For less volatile amines, fractional distillation may be necessary. Final purification of the menthol glycinate is achieve by vacuum distillation.

### Product 4 - (1R,2S,5R)-2-isopropyl-5-methylcyclohexyl 2-(dimethylamino)acetate

Using the general procedure for 2° amines, the (1R,2S,5R)-2-Isopropyl-5-methylcyclohexyl 2-bromoacetate (2) is reacted with dimethylamine (2.0 M solution in THF) to provide the menthol glycinate 4 in 86% yield as clear oil (bp, ca. 140 °C at 1 mm). ¹H NMR (300 MHz, CDCl₃) δ, 0.36-0.42 (m, 2H), 0.42-0.48 (m, 2H), 0.78 (d, 3H, J = 7.0 Hz), 0.85-0.90 (m, 6H), 0.96-1.13 (m, 2H), 1.34-1.44 (m, 1H), 1.46-1.57 (m, 1H), 1.67-1.73 (m, 2H), 1.82-1.90 (m, 1H), 1.97-2.04 (m, 1H), 2.20-2.26 (m, 1H), 2.40 (bs, 1H), 3.34-3.42 (m, 2H), 4.71-4.76 (m, 1H). ¹³C NMR (75 MHz, CDCl₃) δ, 16.2, 20.7, 22.0, 23.3, 26.3, 31.4, 34.2, 40.9, 45.1, 46.9, 60.5, 74.6, 169.9. Low resolution MS (electron impact ionization): 241 (M+), 226, 138, 123, 102.

### Product 8 - (1R,28,5R)-2-Isopropyl-5-methylcyclohexyl 2-aminoacetate

In a deep 3-necked flask, approximately 1.5 mL (0.064 mol) of anhydrous ammonia is condensed at -78 °C and then 7 mL of EtOAc is added. To this solution, NaOH (0.6 g, 15 mmol) is added, followed by dropwise addition of the (1R,2S,5R)-2-Isopropyl-5-methylcyclohexyl 2-bromoacetate (2) as a solution in EtOAc (1.51 g, 5.5 mmol, in 7 mL EtOAc). The solution is stirred for at -60 °C for 2 hours, and then allowed to warm to room temperature, distilling off the excess ammonia. The resulting solution is then filtered through glass wool and the solvent removed with reduced pressure to yield crude 8 as an oil. Spectroscopic data is in accord with previously published data.

Product **8** may be made by an alternative procedure, described as follows. Into a cooled (-78°C) round bottom flask, ammonia (ca. 2 mL, 80 mmol) is condensed and NaOH (0.5 g, 12.5 mmol) is added to the flask. To this flask, an addition funnel is connected. Menthol bromoacetate (compound 2, 1.4 g, 5.0 mmol) is dissolved in 10 mL of ethyl acetate and this solution is placed in the addition funnel. The solution of 2 is then added slowly to the liquid ammonia. The resulting mixture is stirred at -30 °C and monitored by periodically taking samples and subjecting these samples to GCMS analysis. Typically, the conversion is complete within 6 hours. If it is only partially complete, additional ammonia is condensed into the cooled flask. Following completing of the reaction, the mixture is allowed to warm to room temperature and the excess ammonia boils off. To the reaction mixture, anhydrous sodium sulfate is added and the mixture is filtered through a plug of silica gel. The reaction flask is rinsed with 10 mL of ethyl acetate and the solution is passed through the silica gel. The solvent is removed by vacuum to provide a clear oil. Further purification is accomplished by vacuum distillation.

### Product 3 - (1R,2S,5R)-2-isopropyl-5-methylcyclohexyl 2-(diethylamino)acetate

Using the general procedure for 2° amines, the (1R,2S,5R)-2-Isopropyl-5-methylcyclohexyl 2-bromoacetate (2) is reacted with diethylamine to provide the menthol glycinate 3 in 92% yield as clear oil (bp, ca. 160 °C at 1 mm). ¹H NMR (300 MHz, CDCl₃) δ, 0.73 (d, 3H, J = 7.0 Hz), 0.82-0.91 (m, 7H), 0.92-1.2 (m, 1H), 1.05 (t, 6H, J = 7.2 Hz), 1.31-1.40 (m, 1H), 1.40-1.54 (m, 1H), 1.60-1.73 (m, 2H), 1.79-1.89 (m, 1H), 1.94-2.04 (m, 1H), 2.66 (q, 4H, J = 7.2 Hz), 3.30 (s, 2H), 4.67-4.76 (m, 1H). ¹³C NMR (75 MHz, CDCl₃) δ, 9.1, 11.2, 16.0, 20.7, 21.8, 23.1, 26.1, 31.4, 33.8, 40.4, 42.6, 46.5, 56.9, 58.4, 77.8, 164.4. Low resolution MS (electron impact ionization): 269 (M+), 132, 130, 116, 102.

### Product 9 - (1R,2S,5R)-2-Isopropyl-5-methylcyclohexyl 2-(dipropylamino)acetate

Using the general procedure for 2° amines, the (1R,2S,5R)-2-Isopropyl-5-methylcyclohexyl 2-bromoacetate (2) is reacted with dipropylamine to provide the menthol glycinate 9 in 81% yield as clear oil (bp, ca. 170 °C at 1 mm). ¹H NMR (300 MHz, CDCl₃) δ, 0.73 (d, 3H, J = 7.0 Hz), 0.80-0.89 (m, 13H), 0.90-1.08 (m, 1H), 1.30-1.51 (m, 6H), 1.60-1.67 (m, 2H), 1.79-1.88 (m, 1H), 1.92-2.03 (m, 1H), 2.48-2.55 (m, 4H), 3.29 (s, 2H), 4.65-4.74 (m, 1H). ¹³C NMR (75 MHz, CDCl₃) δ, 11.7, 16.2, 20.6, 20.7, 22.0, 23.3, 26.2, 31.3, 34.2, 40.9, 46.9, 55.2, 56.3, 74.1, 171.1. Low resolution MS (electron impact ionization): 297 (M+), 268, 158, 130, 114, 102.

### Product 10 - (1R,2S,5R)-2-Isopropyl-5-methylcyclohexyl 2-(dibutylamino)acetate

Using the general procedure for 2° amines, the (1R,2S,5R)-2-Isopropyl-5-methylcyclohexyl 2-bromoacetate (2) is reacted with dibutylamine to provide the menthol glycinate 10 in 79% yield as clear oil (bp, ca. 180 °C at 1 mm). ¹H NMR (300 MHz, CDCl₃) δ, 0.72 (d, 3H, J = 7.0), 0.83-0.90 (m, 13H), 0.90-1.10 (m, 3H), 1.19-1.53 (m, 9H), 1.61-1.69 (m, 2H), 1.76-1.90 (m, 1H), 1.91-2.01 (m, 1H), 2.54 (t, 4H, J = 7.2), 3.27 (s, 2H), 4.65-4.74 (m, 1H). ¹³C NMR (75 MHz, CDCl₃) δ, 14.0, 16.2, 20.7, 22.0, 23.3, 26.2, 29.6, 31.3, 34.2, 40.9, 47.0, 54.2, 55.2, 74.1, 171.2. Low resolution MS (electron impact ionization): 325 (M+), 282, 144, 143, 142, 102, 100.

### Product 11 - (1R,2S,5R)-2-Isopropyl-5-methylcyclohexyl 2-(pyrrolidin-1-yl)acetate

Using the general procedure for 2° amines, the (1R,2S,5R)-2-isopropyl-5-methylcyclohexyl 2-bromoacetate (2) is reacted with pyrollidine to provide the menthol glycinate 11 in 81% yield as clear oil (bp, ca. 160 °C at 1 mm). ¹H NMR (300 MHz, CDCl₃) δ, 0.75 (d, 3H, J = 7.0), 0.82-0.93 (m, 7H), 0.95-1.11 (m, 2H), 1.33-1.40 (m, 1H), 1.41-1.56 (m, 1H), 1.64-1.72 (m, 2H), 1.76-1.92 (m, 5H), 1.95-2.07 (m, 1H), 2.60-2.70 (m, 4H), 3.25-3.38 (m, 2H), 4.71-4.80 (m, 1H). ¹³C NMR (75 MHz, CDCl₃) δ, 16.3, 20.7, 22.0, 23.4, 23.8, 26.3, 31.4, 34.2, 40.9, 46.9, 53.9, 57.1, 74.3, 170.4. Low resolution MS (electron impact ionization): 267 (M+), 224, 130, 128, 100.

### Product 5 - (1R,2S,5R)-2-isopropyl-5-methylcyclohexyl 2-(piperidin-1-yl)acetate

Using the general procedure for 2° amines, the (1R,2S,5R)-2-isopropyl-5-methylcyclohexyl 2-bromoacetate (2) is reacted with piperidine to provide the menthol glycinate 5 in 61% yield as clear oil (bp, ca. 180 °C at 1 mm). ¹H NMR (300 MHz, CDCl₃) δ, 0.70 (d, 3H, J = 4.2 Hz), 0.72-0.80 (m, 1H), 0.84 (d, 3H, J = 3.6 Hz), 0.86 (d, 3H, J = 3.0 Hz), 0.87-1.0 (m, 3H), 1.34-1.42 (m, 3H), 1.54-1.65 (m, 6H), 1.72-1.86 (m, 1H), 1.90-1.96 (m, 1H), 2.37-2.57 (m, 4H), 3.07 and 3.14 (ABq, 2H, J = 16.5 Hz), 4.61-4.75 (m, 1H). ¹³C NMR (75 MHz, CDCl₃) δ, 16.3, 20.7, 22.0, 23.4, 26.3, 31.4, 34.2, 41.0, 42.2, 46.9, 53.9, 58.2, 74.6, 170.0. Low resolution MS (electron impact ionization): 281 (M+), 266, 144, 142.

### Product 12 - (1R,2S,5R)-2-isopropyl-5-methylcyclohexyl 2-(cyclopropylamino)acetate

Using the general procedure for 1° amines, the (1R,2S,5R)-2-isopropyl-5-methylcyclohexyl 2-bromoacetate (2) is reacted with cyclopropylamine to provide the menthol glycinate **12** in 74% yield as clear oil (bp, ca. 140 °C at 1 mm). ¹H NMR (300 MHz, CDCl₃) δ, 0.36-0.42 (m, 2H), 0.42-0.48 (m, 2H), 0.78 (d, 3H, J = 7.0 Hz), 0.85-0.90 (m, 6H), 0.96-1.13 (m, 2H), 1.34-1.44 (m, 1H), 1.46-1.57 (m, 1H), 1.67-1.73 (m, 2H), 1.82-1.90 (m, 1H), 1.97-2.04 (m, 1H), 2.20-2.26 (m, 1H), 2.40 (bs, 1H), 3.34-3.42 (m, 2H), 4.71-4.76 (m, 1H). ¹³C NMR (75 MHz, CDCl₃) δ, 6.2, 6.3, 16.4, 20.7, 22.0, 23.5, 26.3, 29.9, 31.4, 34.2, 40.9, 47.0, 50.8, 74.7, 172.2. Low resolution MS (electron impact ionization): 253 (M+), 224, 138, 116, 102.

### Product 13 - (1R,2S,5R)-2-isopropyl-5-methylcyclohexyl 2-(bis(2-methoxyethyl)amino)acetate

Using the general procedure for 2° amines, the (1R,2S,5R)-2-isopropyl-5-methylcyclohexyl 2-bromoacetate (2) is reacted with bis(2-methoxyethyl)amine to provide the menthol glycinate **13** in 68% yield as clear oil (bp, ca. 200 °C at 1 mm). ¹H NMR (300 MHz, CDCl₃) δ, 0.69 (d, 3H, J = 7.0), 0.75-0.86 (m, 7H), 0.88-1.07 (m, 2H), 1.25-1.35 (m, 1H), 1.35-1.48 (m, 1H), 1.54-1.66 (m, 2H), 1.73-1.85 (m, 1H), 1.87-1.96 (m, 1H), 2.83-2.90 (m, 4H), 3.24-3.28 (m, 6H), 3.37-3.45 (m, 6H), 4.59-4.71 (m, 1H). ¹³C NMR (75 MHz, CDCl₃) δ, 16.2, 20.7, 22.0, 23.3, 26.2, 31.3, 34.2, 41.0, 46.9, 53.9, 55.9, 58.7, 71.4, 74.1, 171.1. Low resolution MS (electron impact ionization): 297 (M+), 284, 190, 160, 146.

### Product 14 - (1R,2S,5R)-2-Isopropyl-5-methylcyclohexyl 2-(isopentylamino)acetate

Using the general procedure for 1° amines, the (1R,2S,5R)-2-isopropyl-5-methylcyclohexyl 2-bromoacetate (2) is reacted with isopentylamine to provide the menthol glycinate **14** in 90% yield as clear oil (bp, ca. 180 °C at 1 mm). ¹H NMR (300 MHz, CDCl₃) δ, 0.75 (d, 3H, J = 7.0 Hz), 0.87-0.92 (m, 13H), 0.95-1.01 (m, 1H), 1.03-1.13 (m, 1H), 1.35-1.43 (m, 3H), 1.43-1.59 (m, 1H), 1.61-1.74 (m, 3H), 1.77-1.89 (m, 1H), 1.95-2.03 (m, 1H), 2.10 (s, 1H), 2.59-2.64 (m, 2H), 3.38 (s, 2H), 4.70-4.79 (m, 1H). ¹³C NMR (75 MHz, CDCl₃) δ, 16.3, 20.7, 21.9, 22.6, 23.4, 26.0, 26.3, 31.4, 34.2, 38.9, 40.9, 47.0, 47.7, 51.1, 74.7, 171.9. Low resolution MS (electron impact ionization): 283 (M+), 226, 145, 138, 123, 100.

### Product 15 - (1R,2S,5R)-2-isopropyl-5-methylcyclohexyl 2-(benzyl(methyl)amino)acetate

Using the general procedure for 2° amines, the (1R,2S,5R)-2-isopropyl-5-methylcyclohexyl 2-bromoacetate (2) is reacted with N-methylbenzylamine to provide the menthol glycinate **15** in 90% yield as clear oil (bp, ca. 210 °C at 1 mm). ¹H NMR (300 MHz, CDCl₃) δ, 0.78 (d, 3H, J = 7.0 Hz), 0.89 (d, 3H, J = 7.0 Hz), 0.91 (d, 3H, J = 7.0 Hz), 0.96-1.14 (m, 2H), 1.34-1.43 (m, 1H), 1.44-1.57 (m, 1H), 1.64-1.72 (m, 2H), 1.82-1.92 (m, 1H), 1.99-2.05 (m, 1H), 2.39 (s, 3H), 3.24 (s, 2H), 3.69 (s, 2H), 4.73-4.82 (m, 1H). ¹³C NMR (75 MHz, CDCl₃) δ, 16.3, 20.7, 22.0, 23.4, 26.3, 31.4, 34.2, 41.0, 47.0, 57.7, 74.2, 127.1, 128.2, 129.1, 138.4, 170.5. Low resolution MS (electron impact ionization): 317 (M+), 180, 178, 135, 134, 120.

### Product 7 - (1R,2S,5R)-2-Isopropyl-5-methylcyclohexyl 2-(isopropylamino)acetate

Using the general procedure for 1° amines, the (1R,2S,5R)-2-isopropyl-5-methylcyclohexyl 2-bromoacetate (2) is reacted with isopropylamine to provide the menthol glycinate 7 in 88% yield as clear oil (bp, ca. 140 °C at 1 mm). ¹H NMR (500 MHz, CDCl₃) δ, 0.75 (d, 3H, J = 7.0 Hz), 0.84-0.91 (m, 7H), 0.92-1.0 (m, 1H), 1.05 (d, 6H, J = 6.3 Hz), 1.34-1.40 (m, 1H), 1.45-1.53 (m, 1H), 1.64-1.69 (m, 2H), 1.80-1.86 (m, 1H), 1.96- 2.00 (m, 1H), 2.79 (sep, 1H, J = 6.2 Hz), 2.85 (bs, 1H), 3.35-3.39 (m, 2H), 4.70-4.76 (m, 1H). ¹³C NMR (125 MHz, CDCl₃) δ, 16.3, 20.7, 22.0, 22.6, 23.4, 26.3, 31.3, 34.2, 40.9, 47.0, 48.3, 48.8, 48.8, 74.7, 172.2. Low resolution MS (electron impact ionization): 255 (M+), 240, 138, 116, 102.

### Product 16 - (1R,2S,5R)-2-Isopropyl-5-methylcyclohexyl 2-(isopropyl(methyl)amino)acetate

Using the general procedure for 2° amines, the (1R,2S,5R)-2-isopropyl-5-methylcyclohexyl 2-bromoacetate (2) is reacted with methyl-iso-propylamine to provide the menthol glycinate **16** in 88% yield as clear oil (bp, ca. 160 °C at 1 mm). ¹³C NMR (75 MHz, CDCl₃) δ, 16.2, 18.4, 18.4, 20.7, 22.0, 23.4, 26.3, 31.3, 34.2, 38.3, 40.9, 46.9, 53.4, 55.1, 74.3, 171.0. Low resolution MS (electron impact ionization): 269 (M+), 254, 138, 132, 130, 116.

### Product 6 - (1R,2S,5R)-2-Isopropyl-5-methylcyclohexyl 2-(diisobutylamino)acetate

Using the general procedure for 2° amines, the (1R,2S,5R)-2-isopropyl-5-methylcyclohexyl 2-bromoacetate (2) is reacted with diisobutylamine to provide the menthol glycinate 6 in 86% yield as clear oil (bp, ca. 180 °C at 1 mm). ¹H NMR (300 MHz, CDCl₃) δ, 0.74 (d, 3H, J = 7.0), 0.82-0.91 (m, 19H), 0.92-1.11 (m, 2H), 1.30-1.38 (m, 1H), 1.40-1.54 (m, 1H), 1.60-1.71 (m, 4H), 1.81-1.91 (m, 1H), 1.94-2.01 (m, 1H), 2.31 (d, 4H, J = 7.3), 3.26 (s, 2H), 4.65-4.74 (m, 1H). ¹³C NMR (75 MHz, CDCl₃) δ, 16.2, 20.6, 20.7, 22.0, 23.3, 26.2, 26.7, 31.4, 34.2, 41.0, 47.0, 56.0, 63.4, 73.9, 171.6. Low resolution MS (electron impact ionization): 325 (M+), 283, 282, 144, 142, 100.

### Product 17 - (1R,2S,5R)-2-Isopropyl-5-methylcyclohexyl 2-(tert-butylamino)acetate

Using the general procedure for 1° amines, the (1R,2S,5R)-2-isopropyl-5-methylcyclohexyl 2-bromoacetate (2) is reacted with tert-butylamine to provide the menthol glycinate 17 in 78% yield as clear oil (bp, ca. 180 °C at 1 mm). ¹H NMR (300 MHz, CDCl₃) δ, 0.73 (d, 3H, J = 7.0 Hz), 0.86-0.90 (m, 7H), 0.90-1.04 (m, 1H), 1.09 (s, 9H), 1.29-1.37 (m, 1H), 1.38-1.54 (m, 1H), 1.57-1.72 (m, 3H), 1.79-1.92 (m, 1H), 1.94-2.07 (m, 1H), 3.35 (s, 2H), 4.68-4.77 (m, 1H). ¹³C NMR (75 MHz, CDCl₃) δ, 16.3, 20.7, 21.9, 23.4, 26.2, 28.7, 31.3, 34.2, 40.8, 44.9, 47.0, 50.2, 74.6, 172.7. Low resolution MS (electron impact ionization): 269 (M+), 254, 130, 116.

### Product 18 - (1R,2S,5R)-2-Isopropyl-5-methylcyclohexyl 2-(methyl(2-(pyridin-2yl)ethyl)amino)acetate

(1R,2S,5R)-2-isopropyl-5-methylcyclohexyl 2-bromoacetate (2, 1.3 g, 4.69 mmol) is dissolved in 25 mL EtOAc and to this solution is added NaOH (1.2 g, 0.03 mol) and anhydrous Na₂SO₄ (0.5 g). 2-(2-Methylaminoethyl)pyridine (1.0 mL, 7.22 mmol) is added to the solution and the mixture is stirred at room temperature for 4 hours or until GC-FID analysis shows no remaining bromoester 2. The solution is then filtered through glass wool and the solvent removed under reduced pressure. The resulting oil is subjected to vacuum distillation (120 °C at 1 mm for 2 h) to remove excess 2-(2-methylaminoethyl)pyridine. The residue oil is taken up in 20 mL of EtOAc and passed through a plug of SiO₂. Following removal of the solvent, an oil is obtained which is primarily menthol glycinate **18** (1.54 g, 4.63 mmol, 99%). The product is further refined by distillation bp, ca. 230 °C at 1 mm) to provide pure menthol glycinate **18** in 75% yield as clear oil (¹H NMR (300 MHz, CDCl₃) δ, 0.73 (d, 3H, J = 7.0 Hz), 0.80-0.92 (m, 7H), 0.89-1.10 (m, 2H), 1.31-1.43 (m, 1H), 1.43-1.54 (m, 1H), 1.61-1.70 (m, 2H), 1.77-1.89 (m, 1H), 1.94-2.05 (m, 1H), 2.44 (s, 3H), 2.89-3.01 (m, 4H), 3.29 (s, 2H), 4.69-4.78 (m, 1H), 7.06-7.11 (m, 1H), 7.18 (d, 1H, J = 7.8 Hz), 7.54-7.60 (m, 1H), 8.49-8.51 (m, 1H). ¹³C NMR (75 MHz, CDCl₃) δ, 16.2, 20.7, 22.0, 23.4, 26.3, 31.4, 34.2, 36.3, 41.0, 46.9, 56.8, 58.7, 74.4, 121.1, 123.1, 136.3, 149.2, 160.2, 170.5.

### Product 20 - Bis((1R,2S,5R)-2-isopropyl-5-methylcyclohexyl) 2,2'-(isopropylazanediyl)diacetate

(1R,2S,5R)-2-Isopropyl-5-methylcyclohexyl 2-bromoacetate (2, 2.33 g, 8.4 mmol) is dissolved in 25 mL EtOAc and to this solution is added NaOH (1.2 g, 0.03 mol) and anhydrous Na₂SO₄ (0.5 g). isopropylamine (0.25 mL, 2.9 mmol) is added to the solution and the mixture is stirred at room temperature for 5 hours or until GC-FID analysis shows no remaining bromoester **2.** The solution is then filtered through glass wool and the solvent removed under reduced pressure. The resulting oil is subjected to vacuum distillation (160 °C at 1 mm for 20 min) to remove excess bromoester **2.** The distillate is a clear oil (ca. 0.5 g) which is identified as a mixture of bromoester **2** and the desired product **20.** The residue oil is taken up in 20 mL of EtOAc and passed through a plug of SiO₂. Following removal of the solvent, a clear oil is obtained which is menthol glycinate **20** (0.425 g, 0.94 mmol, 32%). ¹H NMR (300 MHz, CDCl₃) δ, 0.76 (d, 6H, J = 6.9 Hz), 0.85-0.95 (m, 14H), 0.94-1.04 (m, 2H), 1.04-1.10 (m, 4H), 1.33-1.42 (m, 2H), 1.42-1.55 (m, 2h), 1.64-1.73 (m, 4H), 1.80-1.93 (m, 2H), 1.96-2.04 (m, 2H), 3.04-3.13 (sep, 1H, J = 6.5 Hz), 3.54 (s, 4H), 4.68-4.77 (m, 2H). ¹³C NMR (75 MHz, CDCl₃) δ, 16.3, 20.3, 20.7, 22.0, 23.4, 26.3, 31.4, 34.3, 40.9, 47.0, 52.7, 74.2, 171.8.

### Product 21 - Bis((1R,2S,5R)-2-isopropyl-5-methylcyclohexyl) 2,2'-(ethane-1,2-diylbis(methylazanediyl))diacetate

(1R,2S,5R)-2-isopropyl-5-methylcyclohexyl 2-bromoacetate (2, 2.13 g, 7.7 mmol) is dissolved in 50 mL EtOAc and to this solution is added NaOH (1.2 g, 0.03 mol) and anhydrous Na₂SO₄ (0.5 g). N,N-dimethylethylenediamine (0.3 mL, 2.79 mmol) is added to the solution and the mixture is stirred at room temperature for 5 hours or until GC-FID analysis shows no remaining bromoester 2. The solution is then filtered through glass wool and the solvent removed under reduced pressure. The resulting oil is subjected to vacuum distillation (170-190 °C at 1 mm for 30 min) to remove excess bromoester **2.** The residue yellow oil is taken up in 20 mL of EtOAc and passed through a plug of SiO₂. Following removal of the solvent, a clear oil is obtained which is menthol glycinate **21** (0.84 g, 1.75 mmol, 43%). ¹H NMR (500 MHz, CDCl₃) δ, 0.78 (d, 6H, J = 6.7 Hz), 0.82-0.95 (m, 14H), 0.96-1.13 (m, 4H), 1.37-1.42 (m, 2H), 1.43-1.55 (bs, 2H), 1.70 (d, 4H, J = 2.4 Hz), 1.80-1.90 (m, 2H), 2.00 (d, 2H, J = 6.9 Hz), 2.48 (s, 6H), 2.82 (s, 4H), 3.42 (s, 4H), 4.74-4.78 (m, 2H). ¹³C NMR (125 MHz, CDCl₃) δ, 16.3, 20.7, 22.0, 23.4, 26.3, 31.4, 34.2, 41.0, 42.2, 46.9, 53.9, 58.2, 74.6, 170.0.

### Example 1: Toxicology review of menthol derivatives

Compounds, referred to by the product numbers used in Table 1, were reviewed for recommended "sip and spit" concentrations, and a preliminary safety evaluation was conducted. Products **3, 5-7, 9-11, 13, 15, 16, 18** and (1R,2S,5R)-2-isopropyl-5-methylcyclohexyl-2-(dibenzylamino)acetate were reviewed. The compounds, as well as potential breakdown compounds were reviewed using toxicology prediction databases. It was determined that samples containing 100 ppm menthol derivatives may be safely tested using the "sip and spit" methodology.

### Example 2: Initial organoleptic evaluation

Solutions were prepared for organoleptic testing using the "sip and spit" method. 100 ppm menthol derivative solutions were prepared by adding 0.025 g of menthol derivative in a 250 mL volumetric flask and with deionized water (DI) at 20 *°C quantum satis* (Q.S.). Solutions were dispersed via agitation prior to tasting. The tasting was conducted by a panel consisting of six participants. Compounds evaluated are: **XI-1-48** (product **9), DK-1-36** (product **5), XI-1-50** (product **16), DK-1-34** (product **6), RK-1-10** (product **15), DK-2-44** (product **11), RK-2-10** (product **10), DK-1-44** (product 13), dimethylamino glycinate (product **4),** and **DK-2-39** (product 7). The tastings revealed a unique property of this class of compounds. Overall, the materials at 100 ppm demonstrated a delayed onset of cooling at 20 seconds to 40 seconds. The compounds demonstrated a very different cooling onset compared to menthol, which provided an instantaneous cooling sensation.

Organoleptic properties by compound are listed below for menthol derivatives in water:
- **XI-1-48** (product **9):** slight estery/fruity aroma with latent onset of cooling.
- **DK-1-36** (product **5):** no cooling present in compound.
- **XI-1-50** (product **16):** no off-notes, onset of cooling at 20 seconds with strong cooling that builds up at 1 minute.
- **DK-1-34** (product **6):** sweet metallic non-offensive note late onset cooling after 20 seconds.
- **RK-1-10** (product **15):** drying mouthfeel sensation, very slight cooling but late onset after 30 seconds.
- **DK-2-44** (product **11):** smells like chlorine with a swimming pool water taste.
- **RK-2-10** (product **10):** delayed onset cool past 20 seconds; very strong cooling profile that extends past two minutes.
- **DK-1-44** (product **13):** bitter, numbness of tongue, slight cooling, more cooling after continuous rinsing with water.
- Product **4:** peppermint-like cooling, very subtle, early cooling.
- **DK-2-39** (product **7):** very delayed cooling past 30 seconds, long lasting post 2 minutes cooling.

### Example 3: Organoleptic evaluation of menthol derivatives in ethanol (EtOH), propylene glycol (PG), and water.

Stock solutions for each solution was prepared as described below to a final concentration of 100 ppm.

### Preparation with ethanol cut in aqueous solution:

### Stock Solution

a. Prepare a stock solution of 2.5% solution in ethanol by adding 25 mg of a menthol analog in 1 gram of ethanol (mix well).
b. Add Q.S. to total weight of 250 grams with DI water.
c. Mix well to achieve final concentration of 100 ppm.

### Preparation with PG cut in aqueous solution:

### Stock Solution

a. Prepare a stock solution of 2.5% solution in PG by adding 25 mg of a menthol analog in 1 gram of PG (mix well).
b. Add Q.S. to total weight of 250 grams with DI water.
c. Mix well to achieve final concentration of 100 ppm.

### Preparation with water:

### Stock Solution

a. Add 25 mg of menthol derivative and DI water Q.S. to total weight of 250 grams to achieve final concentration of 100 ppm.

Organoleptic properties by compound are listed below for menthol derivatives in ethanol, propylene glycol, and water:
- **RK-2-10** (product **10)** exhibited a delayed onset cooling and an upfront bitterness from PG.
- **XI-1-60** (product **7)** sample exhibited late onset cooling after about 10 seconds with a very intense cooling setting in around 40 seconds.
- **XI-1-50** (product **16)** slight delay in cooling after 20 seconds with some upfront bitterness coming from PG. Very intense building cooling post 50 seconds.
- **XI-1-50** (product **16)** PG: initial bitterness from PG cooling at 30 seconds and extending post 1.5 minutes.
- **XI-1-50** (product **16)** EtOH: initial alcohol cooling burn with late onset cooling at 20 seconds and deep cooling at 60 seconds.
- **XI-1-60** (product **7)** PG: Up front cooling at 10 seconds with stronger cooling at 20 seconds, much more intense cooling than XI-1-50.
- **XI-1-60** (product **7)** EtOH: cooling started at 20 seconds with slight alcohol burn, very intense cooling profile from 20 seconds to 2 minutes.
- **RK-2-10** (product **10)** PG: Very low intensity of cooling that slowly builds with a skin tingling effect. Could be a good candidate for taste modification application and bitterness masking at taste threshold use.
- **RK-2-10** (product **10)** EtOH: delay onset mild cooling (post 20 seconds) with a slight tingling and sweetness that extended past 1 minute.

The organoleptic properties of menthol in the simple solutions of PG and water or ethanol were evaluated, and it was found that menthol provided a very strong, instantaneous cooling effect.

### Example 4: Hydrolysis study

Gas chromatography (GC) was used to evaluate the hydrolysis of the menthol derivatives (also referred to as menthol analogs) over time. The average area ratio (area of the composition of interest divided by the area of the internal standard) of the menthol analog and menthol was measured initially, after 24 hours, after 48 hours, after 1 week and after 2 weeks.

The Stock solutions of each menthol derivative in ethanol, PG or water were prepared as described below to a final concentration of 200 ppm. 5 mL of the 200 ppm stock solutions was taken and combined with 1 mL of the internal standard (IS) and added to 10.00 mL chloroform. The organic layer was transferred to the respective GC vials and run. Each GC run was repeated. All stock solutions were stored in the dark at room temperature. All stock solutions were stored in the dark at room temperature.

### Preparation with ethanol cut in aqueous solution:

### Stock Solution

a. Prepare a stock solution of 5% solution in ethanol by adding 50 mg of a menthol analog in 1 gram of ethanol (mix well).
b. Add Q.S. to total weight of 250 grams with DI water.
c. Mix well to achieve final concentration of 200 ppm.

### Preparation with propylene glycol cut in aqueous solution:

### Stock Solution

a. Prepare a stock solution of 5% solution in PG by adding 50 mg of a menthol analog in 1 gram of PG (mix well).
b. Add Q.S. to total weight of 250 grams with DI water.
c. Mix well to achieve final concentration of 200 ppm.

### Preparation in water:

### Stock Solution

a. Prepare a stock solution of 5% solution by adding 50 mg of a menthol analog to DI water.
b. Add DI water Q.S. to total weight of 250 grams.
c. Mix well to achieve final concentration of 200 ppm.

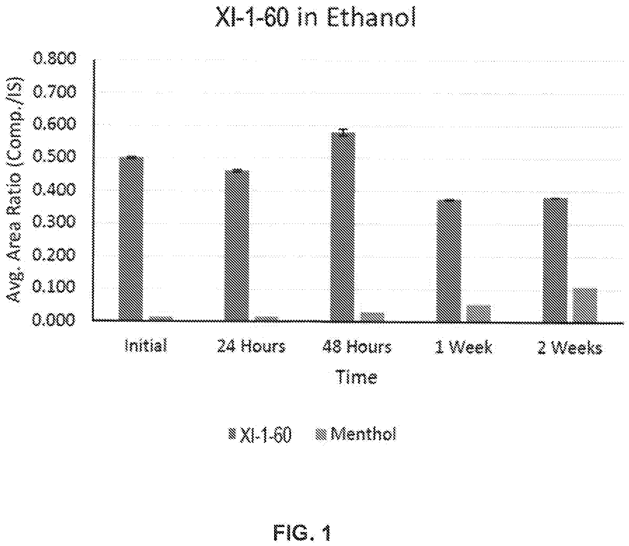

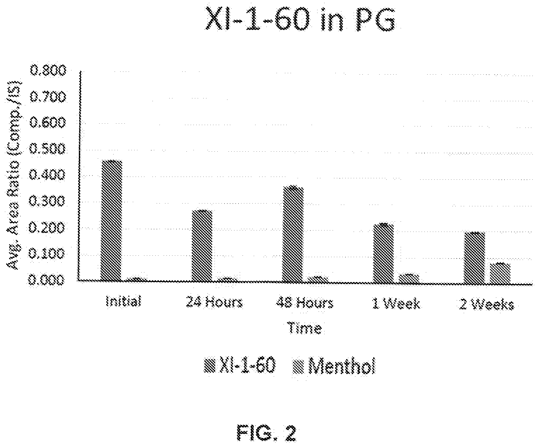

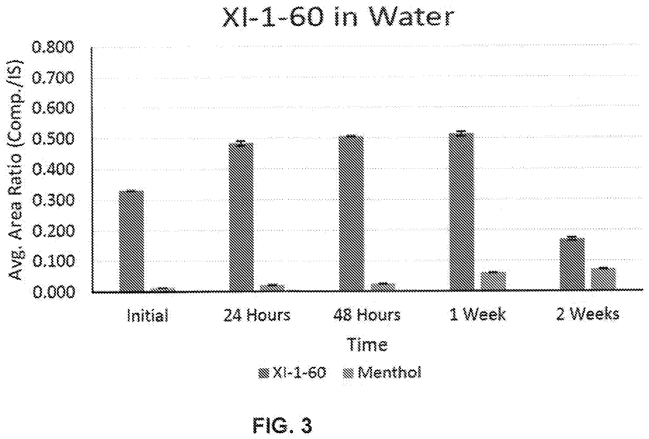

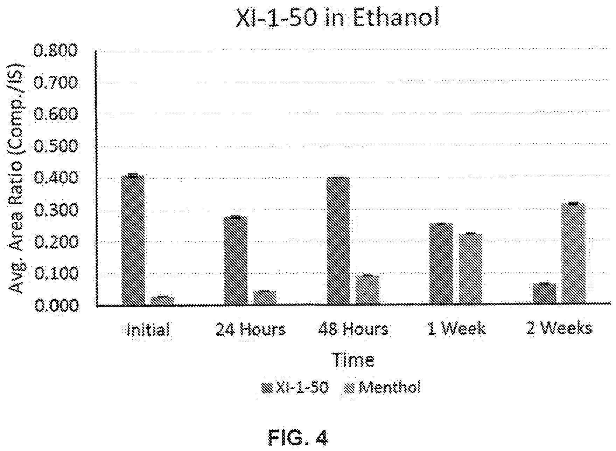

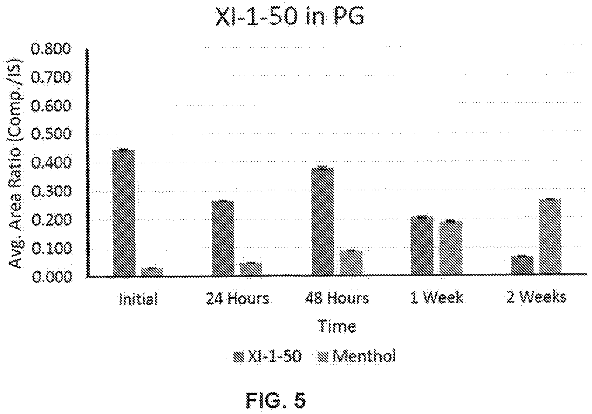

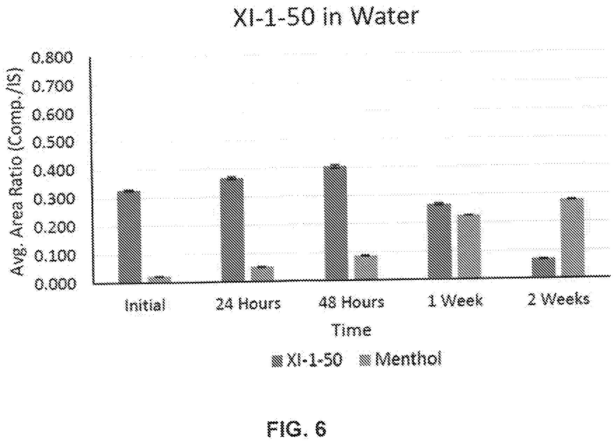

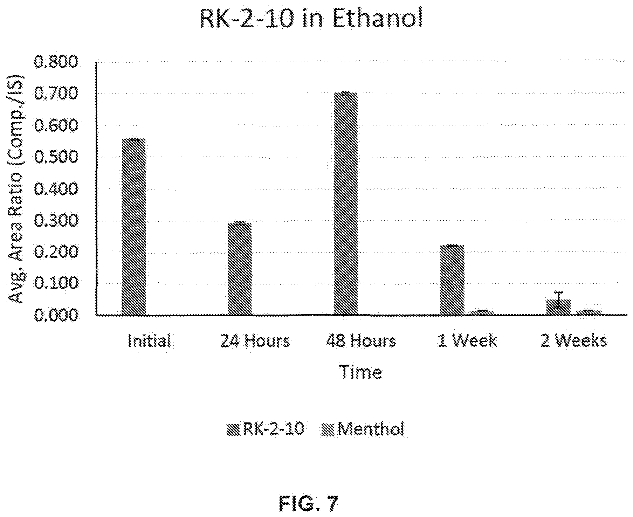

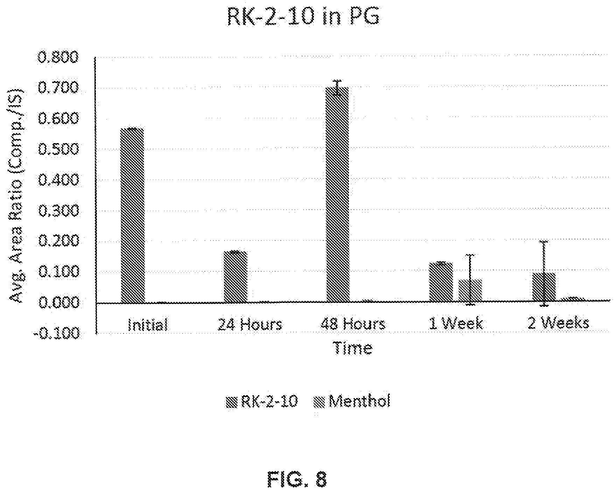

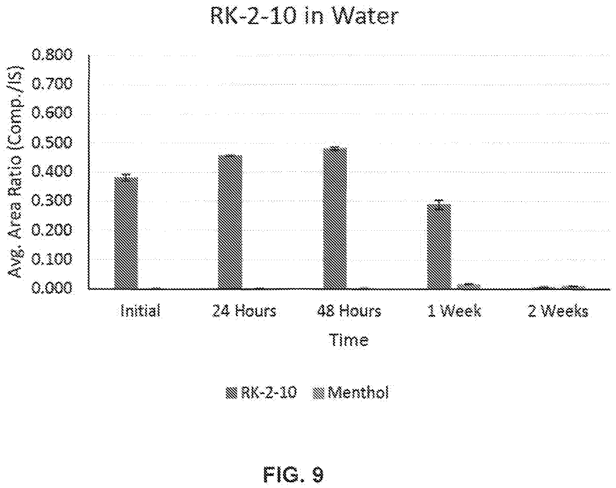

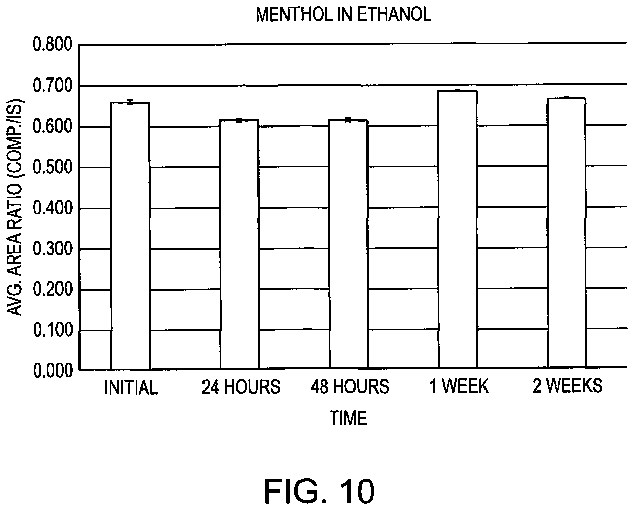

FIG. 1-9 illustrate the average area ratio of the menthol analog and menthol after different periods of time. As the menthol analogs are hydrolyzed, more menthol is present in the solutions. FIG. 10 illustrates the average area ratio of menthol in ethanol.

### Example 5: Solubility analysis

Hansen Solubility Parameters in Practice (HSPiP) was used to calculate Hansen Solubility Parameters for menthol derivatives. Calculation were performed using HSPiP 5^{th} Edition 5.1.02 Licensed to: FONA INTERNATIONAL. Table 3, shown below, displays the data from the Hansen Solubility Parameters for various menthol derivatives and the solvents of interest.

**Table 3: Hansen Solubility Parameters**

| **compound** | **dD** | **dP** | **dH** | **dHD/A** |
|---|---|---|---|---|
| **DK-1-48** (product **9)** | 16.3 | 2.3 | 2.8 | 0.1/3 |
| **DK-1-36** (product **5)** | 17.2 | 2.5 | 3.7 | 0.1/3.5 |
| **XI-1-50** (product **16)** | 16.3 | 2.5 | 2.8 | 0.1/3 |
| **DK-1-34** (product **6)** | 16.1 | 2 | 2.3 | 0.1/2.5 |
| **RK-1-10** (product **16)** | 17.1 | 2.5 | 3.5 | 0.1/3.6 |
| **DK-2-44** (product **11)** | 17.1 | 2.4 | 3.5 | 0.1/3.5 |
| **RK-2-10** (product **10)** | 16.4 | 2.3 | 3 | 0.1/3 |
| **DK-1-44** (product **13)** | 16.5 | 4.2 | 4.5 | 0.1/4.5 |
| Product **4** | 16.4 | 2.8 | 3.5 | 0.1/3.7 |
| **DK-2-39** (product **7)** | 16.6 | 2.7 | 2.9 | 0.6/3.2 |
| water | 15.5 | 16 | 42.3 | 35.4/23.1 |
| I-menthol | 16 | 4.7 | 9 | 5.4/7.2 |
| Propylene Glycol | 16.8 | 10.4 | 21.3 | na |
| Ethanol | 15.8 | 8.8 | 19.4 | 125/11.3 |

### Example 7: Electronic tongue testing example (prophetic)

The compounds of interest were tested using an instrument, referred to as an electronic tongue or e-tongue, for measuring and analyzing flavor profiles. The compounds of interest are tested, and the results are compared to known cooling agents. The electronic tongue has seven sensors that detect the same dissolved organic and inorganic compounds as human taste receptors. Like human receptors, each sensor has a spectrum of reactions different from the other. The information given by each sensor is complementary and the combination of all sensors' results generates a unique fingerprint for the compound to the analyzed.

### Example 8: measuring the time-intensity for menthol and menthol glycinates in lozenges, a beverage model system and a mouthwash

In this example, the experimental compound names correspond to products shown in Table 1. **RK-2-10** corresponds to product **10** from Table 1. **DK-1-50** corresponds to product **16** from Table 1. **XI-1-60** corresponds to product 7 from Table 1. **XI-1-65** corresponds to product 12 from Table 1. **XI-2-73** corresponds to product **15** from Table 1.

In order to evaluate the potential use of menthol glycinates as sensates in different food, beverage and oral hygiene products, select compounds were incorporated in different application systems. A beverage model system, a lozenge and a mouthwash application were created, and time-intensity analysis was performed using trained discrimination analysis panelists.

Time-Intensity (TI) is a temporal sensory method. During TI evaluations, assessors are asked to score the intensity of perception of a single attribute over consumption of the product. Compared to single point measurements, TI analysis can characterize the onset, decline and rates thereof, of particular sensory characteristics. This analysis may reveal rich information regarding the intensity of an attribute over the time domain and identify temporal perceptual differences among samples that can greatly impact the overall profile of a product. For each sample evaluation, a temporal curve is generated, based on duplication. The temporal curves for each panelist are combined to generate a combined curve for each product that is evaluated. The following distinct parameters were calculated:
I ₘₐₓ: peak intensity or maximum observed intensity on the whole curve;
Tₛₜₐᵣₜ: time point where the reaction to the stimulus is first perceived on the curve;
Tₘₐₓ: time position of the peak intensity on the curve;
T ₚₗₐₜₑₐᵤ: protraction of maximum intensity or duration of maximum intensity;
Tₑₓₜ: time point of extinction of the perception of the stimulus, defined as the position in time after the peak intensity where the measured intensity disappears (or the end of evaluation window);
Iₑₓₜ: intensity at time of extinction or end of evaluation;
R increase: slope or rate of intensity increase between Tₛₜₐᵣₜ and Tₘₐₓ;
R decrease: slope or rate of intensity decrease between Tₘₐₓ and Tₑₓₜ; and
Area: the total area under the time-intensity curve.

Details and results regarding the evaluation of select menthol glycinates in applications are presented below.

### Lozenge Application

The formula detailed in Table 4 was employed to create the lozenge application to evaluate menthol glycinates of interest using time-intensity analysis.

**Table 4. Lozenge Formula, ingredients for 100 g batch**

| **Ingredients** | **Amount (g)** |
|---|---|
| Sugar | 88.5 |
| Water | 8.5 |
| Gum Arabic | 2.5 |
| Pork Gelatin, 250 bloom, custom collagen | 0.3 |
| Dementholized corn oil | 0.1 |
| Test Compound* | 0.05 |
| Ethanol | 0.05 |

| | |
|---|---|
| *The following test compounds were evaluated: **RK-2-10,** Menthol, **DK-1-50, XI-1-60** and **XI-1-65.** | |

### Sensory Evaluation of the Lozenge Application

For time-intensity (T-I) evaluation of the cooling intensity of lozenges, 9 panelists were recruited (3 male, 6 female). Panelists were trained in basic taste descriptive analysis. A sweetness reference series (intensity range 2-12) was provided to be used as cross-modality reference to aid in scaling the intensity of the cool attribute present in the lozenge samples. Panelists evaluated samples in duplicates and all samples were presented blinded and in a randomized order using a 3-digit code. Panelists were instructed to provide cooling intensity at predetermined time points throughout an evaluation window of 4 minutes. Samples were presented pre-weighted (2 g) and panelists were instructed to evaluate the entire amount of sample to ensure uniformity among panelists and replicate evaluations. Evaluations were performed with at least 4 hours between them to ensure no carry over.

Data analysis for generation of T-I statistics and ANOVA analysis to determine significant difference among samples was performed using R STUDIO.

### Results for Lozenge Application

All relevant T-I parameters for lozenges are presented in Table 5. Maximum cooling intensity (Iₘₐₓ) values for all sample suggested that all test compounds evaluated have strong cooling potential (cooling range ~9-11.5). ANOVA analysis showed that the maximum cooling intensity of menthol glycinates **XI-1-60** and **XI-1-65** was not significantly different than that of menthol supporting their strong cooling potential. While the cooling maximum intensity of menthol glycinates **RK-2-10** and **DK-1-50** was significantly different to that of menthol overall, their cooling potential was still strong based on the panelist ratings. Tₛₜₐᵣₜ was found to be significantly different for some of the samples evaluated, suggesting a significant difference of the temporal profile and cooling onset. More specifically menthol glycinates **RK-2-10** and **DK-1-50** showed a significantly delayed cooling onset when compared to that of menthol. Menthol glycinate **XI-1-65** had a virtually identical onset to that of menthol. **XI-1-60** exhibited a delayed onset trend, but it was not significantly different to that of menthol. While not all menthol glycinates exhibited a significant cooling onset difference when compared to menthol, all tested menthol glycinates had a significantly delayed Tₘₐₓ when compared to menthol. This shows a significantly different cooling temporal profile. The rate of increase and decrease of cooling intensity also suggests a significant difference between menthol and menthol glycinates. All menthol glycinates trend lower than menthol with **RK-2-10** and **DK-1-50** having significantly lower rates of increase and decrease and **XI-1-60** having a significantly lower rate of decrease suggesting a potentially more prolonged lingering cooling perception.

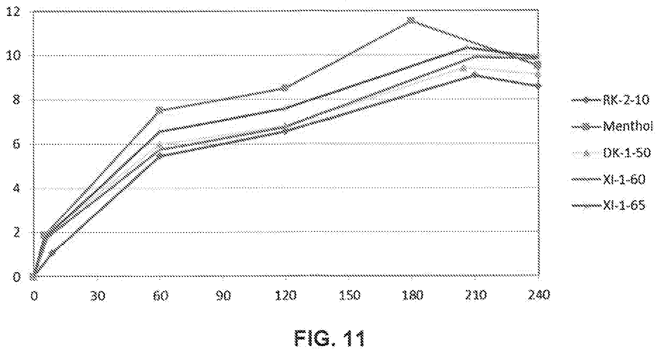

FIG. 11 illustrates a time-intensity graph of the cooling sensation. The total area under the T-I curve (AUC) was also analyzed, correlating with the total perceived cooling intensity within evaluation the time window. Menthol glycinates **XI-1-60** and **XI-1-65** were not significantly different than menthol, while **RK-2-10** and **DK-1-50** were significantly lower.

Overall, menthol glycinates in a lozenge exhibit interesting temporal profiles that suggest their potential use in sensate blends to achieve a more prolonged/lingering cooling impact. With lower cooling increasing and decreasing rate and strong cooling potentials, blend customization can lead to optimized products.

**Table 5. Time-Intensity analysis statistics. Results were obtained using evaluations of lozenges by 9 trained panelists. Lozenges with the following cooling test compounds were evaluated RK-2-10, Menthol, DK-1-50, XI-1-60 and XI-1-65. Each sample was evaluated in duplicates for a 4-minute time window.**

| | **Iₘₐₓ** | **Tₛₜₐᵣₜ** | **Tmax** | **R_{Inc}** | **R_{Doc}** | **AUC** |
|---|---|---|---|---|---|---|
| **RK-2-10** | 9.06^{a} | 8.74a | 210.00⁸ | 0.038^{ac} | 0.038a | 1669.06^{a} |
| **Menthol** | 11.53^{bc} | 5.00^{b} | 180.00^{b} | 0.053b | 0.056^{b} | 2064.83^{b} |
| **DK-1-50** | 9.41^{a} | 7.85^{ac} | 204.71^{a} | 0.039^{ac} | 0.032^{a} | 1698.24^{a} |
| **XI-1-60** | 9.88^{ac} | 6.55^{bc} | 210.00^{a} | 0.041^{ab} | 0.021^{c} | 1750.31^{ab} |
| **XI-1-65** | 10.31^{c} | 5.31^{b} | 206.25^{a} | 0.042^{ab} | 0.042^{ab} | 1945.31^{bc} |

| | | | | | | |
|---|---|---|---|---|---|---|
| Different superscript letters within each column denote statistical significance (ANOVA α=0.5, p<0.05). For variables with the same letter(s), the difference is not statistically significant. Likewise, for variables with a different letter(s), the difference is statistically significant. | | | | | | |

### Beverage Model System

The formula detailed in Table 6 was employed to create a simple beverage model system to evaluate menthol glycinates of interest using time-intensity analysis.

**Table 6. Beverage Model System Formula, ingredients for 100 g batch**

| **Ingredients** | **Amount (g)** |
|---|---|
| Water | 97.99 |
| Propylene Glycol | 2 |
| Test Compound* | 0.01 |

| | |
|---|---|
| *The following test compounds were evaluated: **RK-2-10,** Menthol, **DK-1-50, XI-1-60.** | |

### Sensory Evaluation for Beverage Model System

For time-intensity (T-I) evaluation of the cooling attribute of the model beverage system 8 panelists were recruited (2 male, 6 female). Panelists were trained in basic taste descriptive analysis. A sweetness reference series (intensity range 2-12) was provided to be used as cross-modality reference to aid in scaling the intensity of the cool attribute present in the model beverage samples. Panelists evaluated samples in duplicates and all sample were presented blinded and in a randomized order using a 3-digit code. Panelists were instructed to hold the sample in their mouths for 30 seconds and then expectorate. During the hold period panelists were instructed to note cooling onset and intensity. After expectorating, the panelists continued to report cooling intensity at predetermined time points throughout an evaluation window of 2 minutes. Samples were presented pre-weighted, and panelists were instructed to evaluate the entire amount of sample to ensure uniformity among panelists and replicate evaluations. Evaluations were performed with at least 4 hours between them to ensure no carry over.

Data analysis for generation of T-I statistics and ANOVA analysis to determine significant difference among samples was performed using R STUDIO.

### Results for Beverage Model System

All relevant T-I parameters for beverage model systems are presented in Table 7. Maximum cooling intensity (Iₘₐₓ) values for all samples suggested that all the test compounds that were evaluated have a strong cooling potential, especially in consideration of the low level on incorporation in the model beverage systems (100 ppm). ANOVA analysis showed that the maximum cooling intensity of menthol glycinate **XI-1-60** was not significantly different than that of menthol, supporting the strong cooling potential of that compound. While the cooling maximum intensity of menthol glycinates **RK-2-10** and **DK-1-50** was significantly different to that of menthol overall, their cooling potential was still strong based on the panelists' ratings. Tₛₜₐᵣₜ was found to be significantly different for menthol and all menthol glycinates suggesting significant difference of the temporal profile and cooling onset for all the evaluated novel compounds when compared to menthol. The time to reach maximum cooling intensity was also evaluated and while all menthol glycinates exhibited a trend of higher maximum onset when compared to menthol, only **DK-1-50** was significantly different. This observed trend further supports the different temporal profiles that these menthol glycinates can impart. Plateau time was additionally examined for this evaluation and while menthol was not significantly different than **DK-1-50,** the plateau time for menthol glycinates **RK-2-10** and **XI-1-60** was significantly higher than menthol, suggesting the potential to maintain a peak intensity longer. The rate of increase of cooling intensity also suggests significant differences between menthol and menthol glycinates. All menthol glycinates were found to have a significantly lower rate than menthol, suggesting a potentially more prolonged and lingering cooling perception.

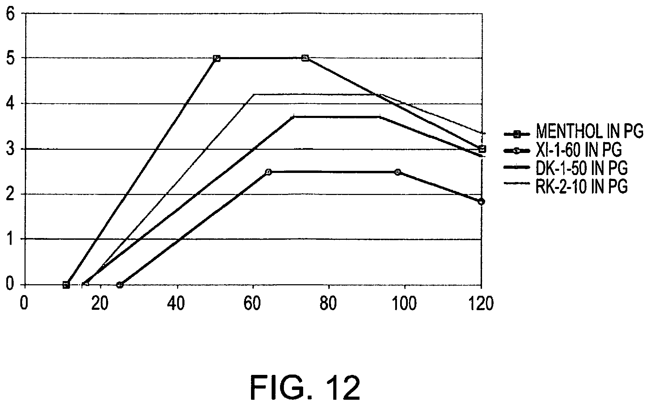

FIG. 12 illustrates a time-intensity graph of the cooling effect in the beverage model system. The total area under the T-I curve was also analyzed correlating with total perceived cooling intensity within evaluation the time window. Menthol glycinate **XI-1-60** was not significantly different than menthol, while **RK-2-10** and **DK-1-50** were significantly lower.

Overall menthol glycinates in a beverage model system exhibit interesting temporal profiles that suggest their potential use in sensate blends to achieve a more prolonged/lingering cooling impact. With lower cooling increasing rates, longer cooling onset, time to reach maximum intensity and strong cooling potentials, blend customization can lead to optimized beverage products.

**Table 7. Time-Intensity analysis statistics. Results were obtained using evaluations of beverage model system by 9 trained panelists. The beverage systems were tested with the following cooling test compounds: RK-2-10, Menthol, DK-1-50, XI-1-60 were evaluated. Each sample was evaluated in duplicates for a 2-minute time window.**

| | **Iₘₐₓ** | **Tₛₜₐᵣₜ** | **Tₘₐₓ** | **Tₚₗₐₜₑₐᵤ** | **R_{Inc}** | **R_{Dec}** | **AUC** |
|---|---|---|---|---|---|---|---|
| **Menthol** | 5^{a} | 10^{a} | 50.2^{a} | 23.3^{a} | 0.12^{a} | 0.03^{a} | 429.1^{a} |
| **RK-2-10** | 2.5^{b} | 6.1^{b} | 58.9^{ab} | 39.6^{b} | 0.04^{b} | 0.06^{a} | 260.1^{b} |
| **DK-1-50** | 3.7^{b} | 16^{c} | 71.6^{b} | 21.8^{a} | 0.05^{b} | 0.02^{a} | 319.6^{bc} |
| **XI-1-60** | 4.2^{ab} | 16^{c} | 60.5^{ab} | 37.2^{b} | 0.06^{b} | 0.02^{a} | 355^{ac} |

| | | | | | | | |
|---|---|---|---|---|---|---|---|
| Different superscript letters within each column denote statistical significance (ANOVA α=0.5, p<0.05). | | | | | | | |

### Mouthwash Application

The formula shown in Table 8 was employed to create a mouthwash product to evaluate methyl glycinates of interest using time-intensity analysis.

**Table 8: Mouthwash Application Formula**

| **Ingredients** | **Amount (g)** |
|---|---|
| Flavor oil | 0.15 |
| Poloxamer 407 | 0.4 |
| Glycerin | 4.5 |
| Sodium lauryl sulfate | 0.3 |
| Na Saccharin | 0.09 |
| NaCl | 0.05 |
| Na Citrate | 0.03 |
| Water | 75 |
| Ethanol | 15 |
| Test Compound | 0.08 |
| Sorbitol | 4.4 |

The following test compounds were evaluated: menthol, **XI-I-60, RK-2-10, XI-1-65, DK-1-50,** and **XI-2-73.**

### Sensory Evaluation for Mouthwash Application

For time-intensity (T-I) evaluation of the cooling attributes of mouthwash products, 8 panelists were recruited (2 male, 6 female). Panelists were trained in basic taste descriptive analysis. A sweetness reference series (intensity range 2-12) was provided to be used as cross-modality reference to aid in scaling the intensity of the cooling attribute present in the mouthwash samples. Panelists evaluated samples in duplicates and all sample were presented blinded and in a randomized order using a 3-digit code. Panelist evaluated 15 ml of mouthwash at a time and were instructed to rinse for 30 seconds and expectorate. Panelists were instructed to provide cooling intensities before and after expectoration. For the time before expectoration, evaluation started upon introducing the sample and every 10 seconds thereafter. After expectoration, the evaluation continued for 15 minutes and panelists provided cooling intensity ratings at predetermined times. Due to the unique nature of this product and the interest in prolonging the cooling attribute long after consumption, an additional parameter Iₑₓₜ was evaluated among samples via ANOVA. This parameter is the cooling intensity at time of extinction or in our case the end of evaluation.

Data analysis for generation of T-I statistics and ANOVA analysis to determine significant difference among samples was performed using R STUDIO.

### Results for Mouthwash Application

All relevant T-I parameters for the mouthwash application are presented in Tables 9a, 9b and 10. Maximum cooling intensity (Iₘₐₓ) values for all sample suggested that all novel menthol glycinates compounds evaluated have strong cooling potential.

Time intensity analysis data for the evaluation of mouthwash samples during the 30 second rinse phase (Table 9a) revealed that all novel glycinates had maximum cooling intensity similar to that of menthol, with glycinates **XI-I-65, DK-1-50,** and **XI-2-73** trending higher, though there was no statistical significance. Time to initial perception (Tₛₜₐᵣₜ), time maximum intensity (Tₘₐₓ), and total area under the curve was also not found to be significantly different among samples during this rinse phase, further supporting the cooling potential of the new glycinates considering the common use of menthol in oral hygiene applications. The R decrease (slope or rate of intensity decrease between Tₘₐₓ and Tₑₓₜ) showed a significant difference between menthol and the menthol glycinates. Menthol glycinates **XI-I-60, RK-2-10, XI-I-65, DK-1-50,** and **XI-2-73** had significantly lower R decrease, with some approaching zero. This suggests that all menthol glycinates maintain their maximum cooling intensity throughout the rinse phase contrary to menthol which starts to decline before expectoration.

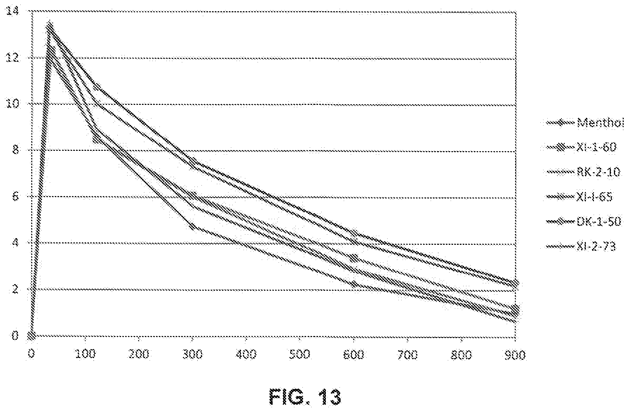

Time intensity analysis for the evaluation of the cooling potential of the novel menthol glycinates in mouthwash application after expectoration (Table 9b) revealed that all novel glycinates had a maximum cooling intensity similar to that of menthol, with all glycinates trending higher when compared to menthol. Although time to initial perception (Tₛₜₐᵣₜ) and time maximum intensity (Tₘₐₓ) were not significantly different among tested compounds, an interesting difference was observed for R increase and R decrease values. R increase (slope or rate of intensity increase between Tₛₜₐᵣₜ and Tₘₐₓ) for menthol glycinates **XI-I-65, RK-2-10,** and **XI-2-73** was positive and significantly higher compared to that of menthol suggesting a continually rising cooling intensity even after expectoration while the same trend was not observed with menthol. This suggests a great cooling potential and the ability for these glycinates to impact the temporal cooling profile in the mouthwash application. In addition, R decrease (slope or rate of intensity decrease between Tₘₐₓ and Tₑₓₜ) for menthol glycinates was significantly lower compared to that of menthol suggesting that menthol glycinates are able to maintain a more intense and lingering cooling sensation after the rinse phase. FIG. 13 illustrates a time-intensity graph of the cooling sensation for the mouthwash. Total areas under the T-I curve also revealed a higher trend for all menthol glycinates when compared to menthol, with **DK-1-50** being significantly higher.

Due to the unique nature of mouthwash products and the interest in prolonging the cooling attribute long after expectoration Iₑₓₜ was evaluated in order to compare the cooling intensity at time of extinction or in our case the end of evaluation among the samples. Results (Table 10) revealed the menthol glycinates **XI-1-65** and **DK-1-50** had significantly higher cooling intensity at the end of our evaluation window when compared to menthol. This observation further supports the potential of these novel sensates in oral hygiene applications to extend cooling attributes. Unique blends of these compounds have the potential to further impart changes to the temporal profile of cooling attributes and thus have great potential in multiple flavoring applications.

Table 9a-9b: Time-Intensity analysis statistics. Results were obtained using evaluations of mouthwash products by 8 trained panelists. Mouthwash products included the following cooling test compounds: menthol, **XI-I-60, RK-2-10, XI-I-65, DK-1-50,** and **XI-2-73.** Each sample was evaluated in duplicates for 2 distinct time windows: rinse phase which lasted 30 seconds (Table 9a) and after expectoration for 15 minutes (Table 9b).

**Table 9a**

| **Product** | **I ₘₐₓ** | **T start** | **T ₘₐₓ** | **R Inc** | **R _{dec}** | **AUC** |
|---|---|---|---|---|---|---|
| **Menthol** | 12.56^{a} | 10.88^{a} | 16.18^{a} | 0.21^{a} | -0.09^{a} | 168.97^{a} |
| **XI-I-60** | 12.43^{a} | 11.00^{a} | 16.33^{a} | 0.20^{a} | -0.02^{b} | 170.50^{a} |
| **RK-2-10** | 12.82^{a} | 10.91^{a} | 15.00^{a} | 0.18^{b} | -0.01b | 182.05^{a} |
| **XI-I-65** | 13.50^{a} | 10.00^{a} | 17.08a | 0.23^{a} | 0.00^{b} | 186.88^{a} |
| **DK-1-50** | 13.09^{a} | 10.00^{a} | 17.27^{a} | 0.23^{a} | 0.00^{b} | 180.91^{a} |
| **XI-2-73** | 13.00^{a} | 10.00^{a} | 17.50^{a} | 0.21^{a} | 0.00^{b} | 179.79^{a} |

| | | | | | | |
|---|---|---|---|---|---|---|
| Different superscript letters within each column denote statistical significance (ANOVA α=0.5, p<0.05). | | | | | | |

**Table 9b**

| **Product** | **I ₘₐₓ** | **T start** | **T max** | **R _{inc}** | **R dec** | **AUC** |
|---|---|---|---|---|---|---|
| **Menthol** | 12.01^{a} | 30.00 ^{a} | 33.53 a | 0.00^{a} | -0.03 a | 3608.38^{a} |
| **XI-I-60** | 12.33^{ab} | 30.00 ^{a} | 36.00 ^{a} | 0.00^{a} | -0.01 b | 4344.50^{ac} |
| **RK-2-10** | 12.65^{ab} | 30.00 a | 30.00 ^{a} | 0.00 a | -0.01 b | 4105.91^{ac} |
| **XI-I-65** | 13.20ab | 30.00 ^{a} | 32.50 ^{a} | 0.03 ^{b} | -0.01 b | 5211.25^{bc} |
| **DK-1-50** | 13.37^{ab} | 30.00 ^{a} | 32.73 a | 0.03 ^{b} | -0.01 b | 5565.00^{b} |
| **XI-2-73** | 13.50^{ab} | 30.00 ^{a} | 32.50 ^{a} | 0.03 ^{b} | -0.01 b | 4082.50^{ac} |

| | | | | | | |
|---|---|---|---|---|---|---|
| Different superscript letters within each column denote statistical significance (ANOVA α=0.5, p<0.05). | | | | | | |

**Table 10. Cooling intensity at time of extinction or the end of evaluation. Mouthwash products evaluated the following cooling test compounds: menthol, XI-I-60, RK-2-10, XI-I-65, DK-1-50, and XI-2-73. Each sample was evaluated in duplicates and the parameter is focused on cooling intensity reported at 15 minutes after expectoration.**

| **Product** | **Iₑₓₜ** |
|---|---|
| **Menthol** | 1^{a} |
| **XI-I-60** | 1.23^{a} |
| **RK-2-10** | 0.9^{a} |
| **XI-I-65** | 2.18^{b} |
| **DK-1-50** | 2.33^{b} |
| **XI-2-73** | 0.67^{a} |

| | |
|---|---|
| Different superscript letters within each column denote statistical significance (ANOVA α=0.5, p<0.05). | |

### REFERENCES

(1) Eccles, R. (1994). "Menthol and Related Cooling Compounds". J. Pharm. Pharmacol. 46 (8): 618-630.
(2) "Update on Menthol Production & Use." http://www .leffingwell.com/menthol1/menthol1.htm; downloaded 12/31/18.
(3) "Cool without Menthol & Cooler than Menthol and Cooling Compounds as Insect Repellents." Leffingwell, J. C., http://www.leffingwell.com/cooler_than_menthol.htm; 12/31/18.
(4) "Common Fragrance and Flavor Materials: Preparation, Properties and Uses 4th Ed." Bauer, K.; Garbe, D.; Surburg, H., Wiley-VHC, Weinheim, Germany, 2001.
(5) Hettstedt, C.; Betzl, W.; Karaghiosoff, K. Zeits. Anorg. Allgem. Chemie 2012, 638, 377. For alternative routes leading to compound 2, see:
(6) Shankar, S. P.; Jagodzinska, M.; Malpezzi, L.; Lazzari, P.; Manca, I.; Greig, I. R.; Sani, M.; Zanda, M. Org. Biomol. Chem. 2013, 11, 2273.
(7) Singh, R.; Ghosh, S. K. Tetrahedron: Asymmetry, 2014, 25, 57. d. Streinz, L.; Koutek, B.; Saman, D. Synlett, 2001, 809.
(8) Dentel, H.; Chataigner, I.; Lohier, J.-F.; Gulea, M. Tetrahedron, 2012, 68, 2326.
(9) Yin, Y.; Wu, M.; Zubcevic, L.; Borschel, W. F.; Lander, G. C.; Lee, S.-Y. Science, 2018, 359, 237.
(10) Shirai, T.; Kumihashi, K.; Sakasai, M. Kusuoku, H.; Shibuya, Y.; Ohuchi, A. ACS Med. Chem. Lett. 2017, 8, 715.
(11) Kweka, E.J., et al., Protective efficacy of menthol propylene glycol carbonate compared to N,N-diethyl-methylbenzamide against mosquito bites in Northern Tanzania, Parasites & Vectors, 5:189 (2012)
(12) Hansen Solubility Parameters in Practice, available at https://www.hansen-solubility.com/HSPiP/
(13) "Synthesis of Menthol Glycinates and Their Potential as Cooling Agents." Klumpp, D. A.; Sobel, R. M.; Osei-Badu, B.; Liveras, Z.; Klumpp, R. A.; Kokkinidou, S. G.; Stentzel, M. R. ACS Omega, 2020, 5, 4043-4049 (doi.org/10.1021/acsomega.9b03624).

## Claims

1. A method for providing a cooling sensation in the mouth, comprising: orally administering a cooling agent, wherein the cooling agent is a compound of Formula (I) 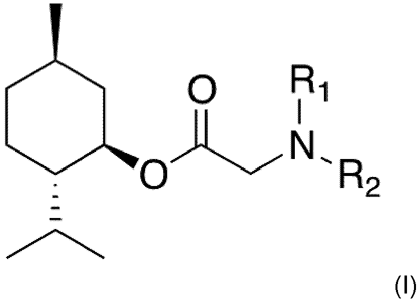
R₁ and R₂ are independently selected from the group consisting of: H, alkyl and R₁ and R₂ together with the N atom to which they are attached form a 4-8-member ring, wherein alkyl is defined as a monovalent, substituted or unsubstituted, saturated or unsaturated, straight, branched, or cyclic hydrocarbon chain; wherein substituents are selected from the group consisting of hydroxyl, amino, oxy, carbonyl, thiol, alkyl, alkoxy, halo, nitrile, nitro, aryl, and heterocyclic groups; and
R₁ and R₂ each contain at most 20 carbon atoms.

2. A compound of Formula (II) 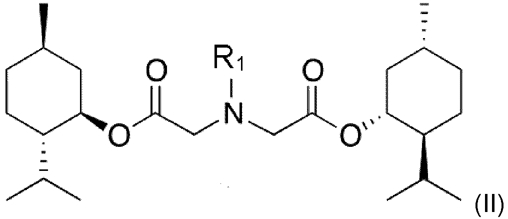 or Formula (III) 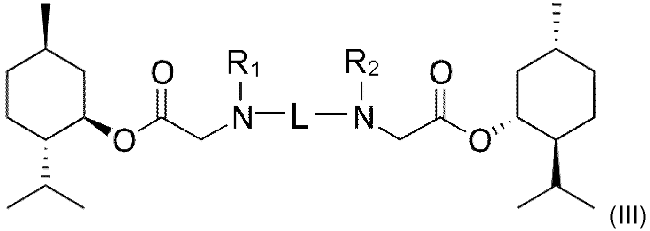
wherein R₁ and R₂ are independently selected from the group consisting of: H, alkyl and R₁ and R₂ together with the N atom to which they are attached form a 4-8-member ring, wherein alkyl is defined as a monovalent, substituted or unsubstituted, saturated or unsaturated, straight, branched, or cyclic hydrocarbon chain; wherein substituents are selected from the group consisting of hydroxyl, amino, oxy, carbonyl, thiol, alkyl, alkoxy, halo, nitrile, nitro, aryl, and heterocyclic groups;
R₁ and R₂ each contain at most 20 carbon atoms, and
L is an alkylene group, arylene group, -(CH₂)_{X}-(OCH₂CH₂)ₙO-(CH₂)_{X}- where x and n are individually 1 to 10 and x + n is at most 10, or 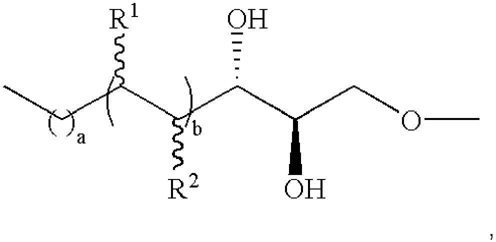 where R¹ and R² are each individually selected from the group consisting of H and OH and a and b are individually 0, 1, 2 or 3, where the L group has at most 20 carbon atoms.

3. The method of claim 1 or the compound of claim 2, wherein at least one of R₁ and R₂ is (i) a substituted alkyl, preferably a group comprising a member selected from the group consisting of an aryl group, an ether group and an amine group, or (ii) an alkyl group of 3-6 carbon atoms, preferably a propyl group.

4. The method of claim 1 or the compound of claim 2, wherein R₁ is selected from the group consisting of: a methyl group, an ethyl group and a hydrogen atom.

5. The method of claim 1 or the compound of claim 2, wherein R₁ comprises at least one group selected from the group consisting of: piperidine and pyridine.

6. The method of claim 1 or the compound of claim 2, wherein (i) R₁ and R₂ and the N atom to which they are attached form a piperidine or pyrrolidine ring, or (ii) both R₁ and R₂ comprise an alkyl group of 3-6 carbon atoms.

7. The method of any one of claims 1 or 3-6, wherein the cooling agent is provided in a form selected from the group consisting of: a candy, gum, beverage, mouthwash and toothpaste.

8. A method of repelling insects, comprising: applying the compound of any one of claims 2-6.

9. A skin cream or shave gel, comprising the compound of any one of claims 2-6 and a carrier.

10. The compound of any one of claims 2-6, wherein L is selected from the group consisting of methylene, (CH₂)₂, (CH₂)₃, (CH₂)₄, (CH₂)(CH)(CH₃), and (CH₂)(CH₂)(CH)(CH₃).

11. A method for providing a cooling sensation in the mouth, comprising: orally administering a cooling agent, wherein the cooling agent is the compound of any one of claims 2-6 or 10.

12. An oral hygiene product or an edible product, comprising: the compound of any one of claims 2-6 or 10.

## Patentansprüche

1. Verfahren zum Bereitstellen eines kühlenden Gefühls im Mund, umfassend: orales Verabreichen eines kühlenden Mittels, wobei das kühlende Mittel eine Verbindung der Formel (I) ist 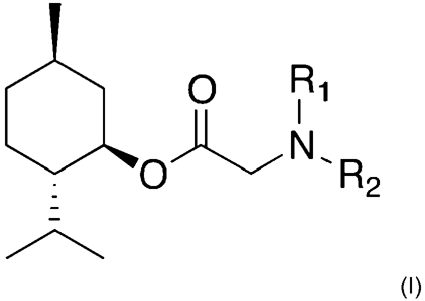 R₁ und R₂ unabhängig voneinander ausgewählt sind aus der Gruppe, bestehend aus: H, Alkylgruppe, und R₁ und R₂ zusammen mit dem N-Atom, an das sie gebunden sind, einen 4-8-gliedrigen Ring bilden, wobei die Alkylgruppe als eine monovalente, substituierte oder unsubstituierte, gesättigte oder ungesättigte, gerade, verzweigte oder cyclische Kohlenwasserstoffkette definiert ist; wobei Substituenten aus der Gruppe ausgewählt sind, die aus Hydroxyl-, Amino-, Oxy-, Carbonyl-, Thiol-, Alkyl-, Alkoxy-, Halogen-, Nitril-, Nitro-, Aryl- und heterocyclischen Gruppen besteht; und R₁ und R₂ jeweils höchstens 20 Kohlenstoffatome enthalten.

2. Verbindung der Formel (II) 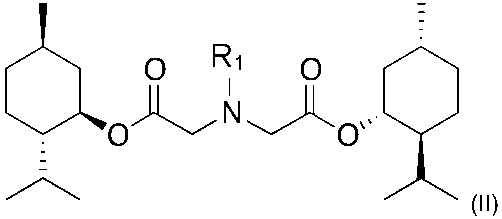 oder der Formel (III) 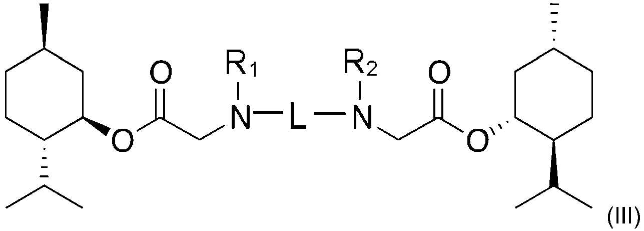
worin R₁ und R₂ unabhängig voneinander ausgewählt sind aus der Gruppe, bestehend aus: H, Alkylgruppe, und R₁ und R₂ zusammen mit dem N-Atom, an das sie gebunden sind, einen 4-8-gliedrigen Ring bilden, wobei die Alkylgruppe als eine monovalente, substituierte oder unsubstituierte, gesättigte oder ungesättigte, gerade, verzweigte oder cyclische Kohlenwasserstoffkette definiert ist; wobei Substituenten aus der Gruppe ausgewählt sind, die aus Hydroxyl-, Amino-, Oxy-, Carbonyl-, Thiol-, Alkyl-, Alkoxy-, Halogen-, Nitril-, Nitro-, Aryl- und heterocyclischen Gruppen besteht; R₁ und R₂ jeweils höchstens 20 Kohlenstoffatome enthalten, und
L eine Alkylengruppe, Arylengruppe, -(CH₂)_{X}-(OCH₂CH₂)ₙO-(CH₂)_{X}-, wobei x und n individuell 1 bis 10 sind und x + n höchstens 10 ist, oder 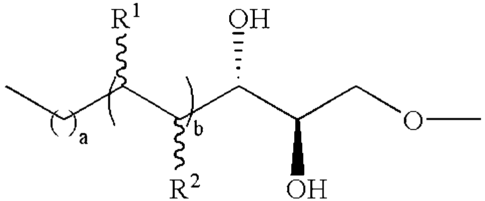 ist, worin R¹ und R² jeweils individuell aus der Gruppe ausgewählt sind, die aus H und OH besteht, und a und b individuell 0, 1, 2 oder 3 sind, wobei die L-Gruppe höchstens 20 Kohlenstoffatome aufweist.

3. Verfahren nach Anspruch 1 oder Verbindung nach Anspruch 2, wobei mindestens einer der Reste R₁ und R₂ (i) eine substituierte Alkylgruppe, vorzugsweise eine Gruppe, die ein Mitglied umfasst, ausgewählt aus der Gruppe, bestehend aus einer Arylgruppe, einer Ethergruppe und einer Amingruppe, oder (ii) eine Alkylgruppe mit 3-6 Kohlenstoffatomen, vorzugsweise eine Propylgruppe, ist.

4. Verfahren nach Anspruch 1 oder Verbindung nach Anspruch 2, wobei R₁ aus der Gruppe ausgewählt ist, bestehend aus: einer Methylgruppe, einer Ethylgruppe und einem Wasserstoffatom.

5. Verfahren nach Anspruch 1 oder Verbindung nach Anspruch 2, wobei R₁ mindestens eine Gruppe umfasst, die aus der Gruppe ausgewählt ist, bestehend aus: Piperidin und Pyridin.

6. Verfahren nach Anspruch 1 oder Verbindung nach Anspruch 2, wobei (i) R₁ und R₂ und das N-Atom, an das sie gebunden sind, einen Piperidin- oder Pyrrolidinring bilden, oder (ii) beide R₁ und R₂ eine Alkylgruppe mit 3-6 Kohlenstoffatomen umfassen.

7. Verfahren nach einem jeglichen der Ansprüche 1 oder 3 bis 6, wobei das kühlende Mittel in einer Form bereitgestellt wird, die ausgewählt ist aus der Gruppe, bestehend aus: einer Süßigkeit, einem Gummi, Getränk, Mundwasser und einer Zahnpasta.

8. Verfahren zum Schutz vor Insekten, umfassend: Auftragen der Verbindung nach einem jeglichen der Ansprüche 2 bis 6.

9. Hautcreme oder Rasiergel, umfassend die Verbindung nach einem jeglichen der Ansprüche 2 bis 6 und einen Träger.

10. Verbindung nach einem jeglichen der Ansprüche 2 bis 6, wobei L ausgewählt ist aus der Gruppe, bestehend aus Methylengruppe, (CH₂)₂, (CH₂)₃, (CH₂)₄, (CH₂)(CH)(CH₃) und (CH₂)(CH₂)(CH)(CH₃).

11. Verfahren zum Bereitstellen eines kühlenden Gefühls im Mund, umfassend: orales Verabreichen eines kühlenden Mittels, wobei das kühlende Mittel die Verbindung nach einem jeglichen der Ansprüche 2 bis 6 oder 10 ist.

12. Mundhygieneprodukt oder essbares Produkt, umfassend: die Verbindung nach einem jeglichen der Ansprüche 2 bis 6 oder 10.

## Revendications

1. Procédé destiné à procurer une sensation rafraîchissante dans la bouche, comprenant : l'administration par voie orale d'un agent rafraîchissant, ledit agent rafraîchissant étant un composé de Formule (I) 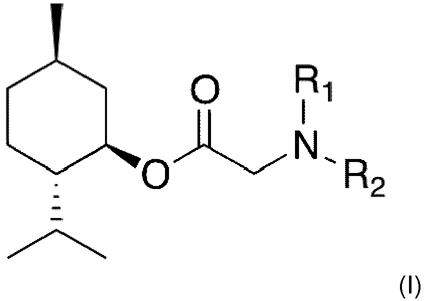
R₁ et R₂ sont indépendamment choisis dans le groupe constitué de : H, alkyle et R₁ et R₂ conjointement avec l'atome N auquel ils sont liés forment un cycle à 4 à 8 chaînons, ledit alkyle étant défini comme une chaîne hydrocarbonée monovalente, substituée ou non substituée, saturée ou insaturée, linéaire, ramifiée ou cyclique ; dans lequel les substituants sont choisis dans le groupe constitué des groupes hydroxyle, amino, oxy, carbonyle, thiol, alkyle, alkoxy, halogéno, nitrile, nitro, aryle et hétérocyclique ; et
R₁ et R₂ contiennent chacun au plus 20 atomes de carbone.

2. Composé de Formule (II) 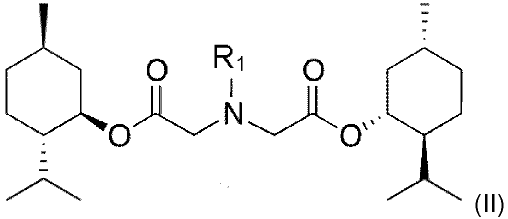 ou de Formule (III) 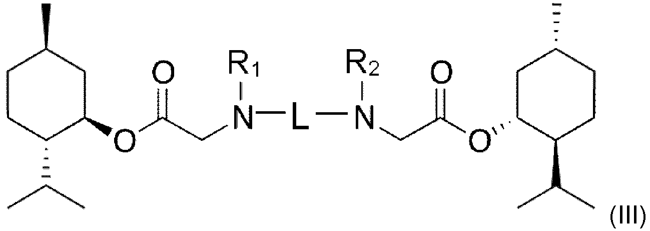
dans lequel R₁ et R₂ sont indépendamment choisis dans le groupe constitué de : H, alkyle et R₁ et R₂ conjointement avec l'atome N auquel ils sont liés forment un cycle à 4 à 8 chaînons, ledit alkyle étant défini comme une chaîne hydrocarbonée monovalente, substituée ou non substituée, saturée ou insaturée, linéaire, ramifiée ou cyclique ; dans lequel les substituants sont choisis dans le groupe constitué des groupes hydroxyle, amino, oxy, carbonyle, thiol, alkyle, alkoxy, halogéno, nitrile, nitro, aryle et hétérocyclique ;
R₁ et R₂ contiennent chacun au plus 20 atomes de carbone, et
L est un groupe alkylène, un groupe arylène, -(CH₂)_{X}-(OCH₂CH₂)ₙO-(CH₂)_{X}-, où x et n sont individuellement 1 à 10 et x + n est au plus 10,
ou 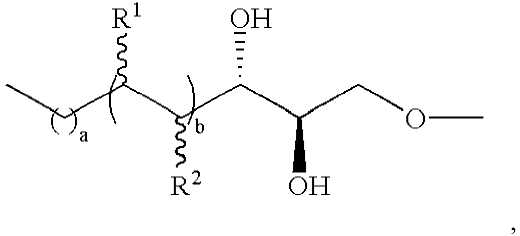 R₁ et R₂ sont chacun individuellement choisis dans le groupe constitué de H et OH, et a et b sont individuellement 0, 1, 2 ou 3, où le groupe L comporte au plus 20 atomes de carbone.

3. Procédé de la revendication 1 ou composé de la revendication 2, dans lequel au moins l'un de R₁ et R₂ est (i) un alkyle substitué, de préférence un groupe comprenant un élément choisi dans le groupe constitué d'un groupe aryle, un groupe éther et un groupe amine, ou (ii) un groupe alkyle de 3 à 6 atomes de carbone, de préférence un groupe propyle.

4. Procédé de la revendication 1 ou composé de la revendication 2, dans lequel R₁ est choisi dans le groupe constitué de : un groupe méthyle, un groupe éthyle et un atome d'hydrogène.

5. Procédé de la revendication 1 ou composé de la revendication 2, dans lequel R₁ comprend au moins un groupe choisi dans le groupe constitué de : pipéridine et pyridine.

6. Procédé de la revendication 1 ou composé de la revendication 2, dans lequel (i) R₁ et R₂ et l'atome N auquel ils sont liés forment un cycle pipéridine ou pyrrolidine, ou (ii) R₁ et R₂ comprennent tous deux un groupe alkyle de 3 à 6 atomes de carbone.

7. Procédé de l'une quelconque des revendications 1 ou 3 à 6, dans lequel l'agent rafraîchissant est fourni sous une forme choisie dans le groupe constitué de : un bonbon, une gomme, une boisson, un bain de bouche et un dentifrice.

8. Procédé pour repousser les insectes, comprenant : l'application du composé de l'une quelconque des revendications 2 à 6.

9. Crème pour la peau ou gel de rasage, comprenant le composé de l'une quelconque des revendications 2 à 6 et un support.

10. Composé de l'une quelconque des revendications 2 à 6, dans lequel L est choisi dans le groupe constitué de méthylène, (CH₂)₂, (CH₂)₃, (CH₂)₄, (CH₂)(CH)(CH₃) et (CH₂)(CH₂)(CH)(CH₃).

11. Procédé destiné à procurer une sensation rafraîchissante dans la bouche, comprenant : l'administration par voie orale d'un agent rafraîchissant, ledit agent rafraîchissant étant le composé de l'une quelconque des revendications 2 à 6 ou 10.

12. Produit d'hygiène buccale ou produit comestible, comprenant : le composé de l'une quelconque des revendications 2 à 6 ou 10.
